# EUROPEAN PATENT APPLICATION

(11) **EP 4 095 246 A1**
(43) Date of publication of application: **30.11.2022**
(21) Application number: 21176366.9
(22) Date of filing: 27.05.2021
(51) Int. Cl.: C12N 15/11, C12N 15/113

(54) **NOVEL CRISPR GRNAS**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention relates to a guide RNA (gRNA) suitable for CRISPR-mediated oligonucleotide binding and/or editing comprising at least one hairpin that does not interact with a Cas enzyme wherein said hairpin forms a locked secondary structure.

## Description

The present invention relates to a guide RNA (gRNA) suitable for CRISPR-mediated oligonucleotide binding and/or editing comprising at least one hairpin that does not interact with a Cas enzyme wherein said hairpin forms a locked secondary structure.

### Background

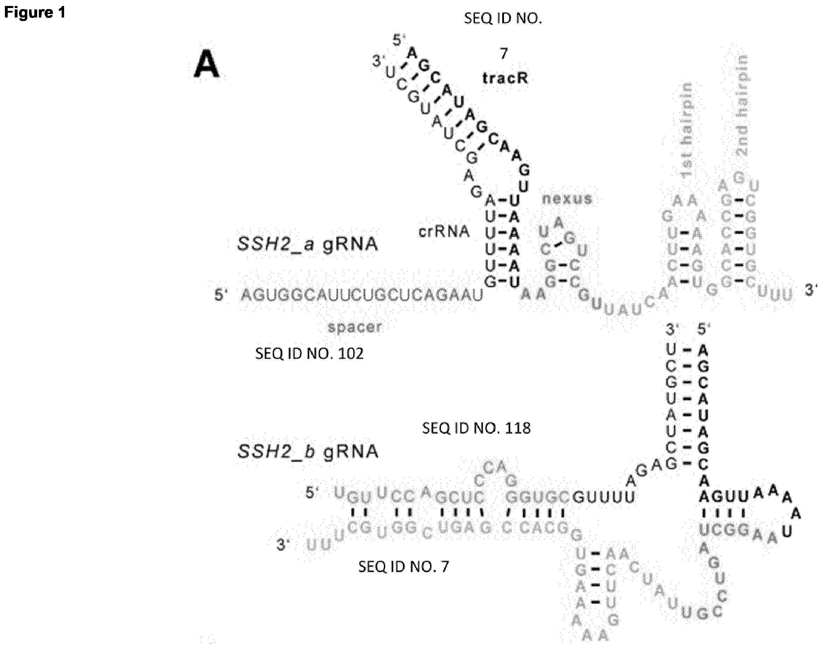

The CRISPR-Cas system, in particular the CRISPR-Cas9 system is an invaluable tool for genome modification. A Cas nuclease such as Cas9 can introduce a DNA double-strand break (DSB) in a sequence target that is complementary to a 20-nt spacer sequence of its bound guide RNA (gRNA) when a protospacer adjacent motif (PAM) is present. The gRNA can be provided as duplex of spacer containing CRISPR RNA (crRNA) and trans-activating crRNA (tracrRNA) or as a single gRNA (sgRNA) where crRNA and tracrRNA are fused by an artificial loop (Jinek et al., 2012). A gRNA consists of the target-specific spacer and a constant part typically comprised of distinct motifs including the nexus, and a first and second hairpin (Briner et al., 2014) (Fig. 1A). Cellular repair of targeted DNA DSBs by error-prone end-joining pathways often lead to frameshift mutations that enable knockout studies of genes of interest and DNA donor enabled homology-directed repair (HDR) allows precise control of the inserted mutation.

One widely used approach to increase gRNA cleavage and thus genome editing efficiency is the use of chemical modifications in synthesized gRNA, preferably 5'- and 3'-end protection using phosphorothioate bonds and internal 2'-OMe residues, to improve its stability against degradation by nucleases (Hendel et al., 2015; Yin et al., 2017). Genome editing efficiencies can be further increased by providing the cell with external non-homologous DNA and it is speculated that this non-homologous oligonucleotide enhancement (NOE) diverts cells towards error-prone instead of error-free repair pathways (Richardson et al., 2016). This effect is also achieved when using DNA as electroporation enhancer or providing a DNA donor as a repair template for HDR. Elongated crRNA/tracR hybridization as present in the bacterial CRISPR target complex, and an A-T flip that removes the TTTT transcription termination signal, improve genome editing compared to standard *in-vivo* transcribed sgRNA (Chen et al., 2013). These modifications are combined into Hybridization extended A-T inversion (HEAT) gRNA. Improved Cas9 activity on certain target sequences was achieved by sequence alterations, i.e. deletion and/or replacement of uridines in the linker region (34A) and in the first hairpin (U39G-U40G) of the tracrRNA (Scott et a., 2019 and PCT/US2018/06605).

On the other hand, Anderson et al. (2018) describe that an elongation of the repeat-anti-repeat duplex in the *Lactobacillus gasseri* Cas9 gRNA leads to a reduction of genome editing efficacy.

Further, genome editing efficiencies vary strongly from target to target and some targets are totally intractable to genome editing even when chemically stabilized gRNAs, HEAT gRNAs, and NOE are employed. This results in a need to pre-screen several gRNAs to find an efficient one, which is worsened by frequent discrepancy of *in-vitro* cleavage efficiency and *in-vivo* genome editing efficiency (Briner et al., 2014). Numerous gRNA efficiency algorithms have been employed to allow *in-silico* pre-screening, but their accuracy is far from satisfying (Wilson et al., 2018). Also, investigation of certain biological questions or repair of disease alleles can be limited to the use of a potentially inefficient single target site.

It has been described that gRNAs can be inactive due to internal misfolding and that they can compete with active gRNAs for binding to Cas9 (Thyme et al., 2016). Underlining importance of self-folding, a deep machine-learning model trained on activity data of over 50.000 gRNAs revealed that self-folding free energy strongly influences cleavage efficiency and can have the strongest impact of 1031 tested features on the model output (Wang et al., 2019a). Further, low terminal melting temperature (in spacer residues 1-4 and 16-20) can reduce cleavage efficiencies (Wang et al., 2019a) and PAM-proximal TT- or GCC-motifs have been described to block editing (Graf et al., 2019).

It is an objective of the present invention solve the problem of low or absent genome editing by providing a modified gRNA, particularly a modified tracrRNA, which allows robust genome editing regardless of spacer sequence composition.

### Summary of the Invention

The present inventors have solved the problem of low or absent genome editing by providing a modified gRNA, particularly a modified tracrRNA with an unusually stable hairpin, which prevents misfolding of the gRNA, and optionally comprises at least one modified nucleotide building block. This novel genome editing optimized locked design (GOLD) - gRNA allows robust genome editing regardless of spacer sequence composition.

The gRNA may be a single guide RNA (sgRNA) molecule or plurality of RNA molecules, particularly comprising a spacer-containing CRISPR RNA (crRNA) molecule and a trans-activating CRISPR RNA (tracrRNA) molecule.

A first aspect of the present invention relates to a guide RNA (gRNA) suitable for CRISPR-mediated Oligonucleotide binding and/or editing, comprising:
- a target-specific spacer sequence and
- a constant gRNA sequence comprising at least one locked hairpin secondary structure at a position that does not interfere with Oligonucleotide binding and/or editing.

A further aspect of the present invention relates to a gRNA suitable for CRISPR-mediated Oligonucleotide binding and/or editing comprising:
- a target-specific spacer sequence and
- a constant gRNA sequence comprising a CRISPR repeat/anti-repeat-structure or a portion thereof and at least one locked hairpin secondary structure at a position that does not interfere with Oligonucleotide binding and/or editing.

A further aspect of the present invention relates to a gRNA suitable for CRISPR/Cas9-mediated Oligonucleotide binding and/or editing comprising in 5' to 3' direction:
- a target-specific spacer sequence and
- a constant gRNA sequence comprising in 5' to 3' direction a (i) repeat/anti-repeat structure, (ii) optionally a nexus sequence and (iii) at least one locked hairpin secondary structure.

A further aspect of the present invention relates to a gRNA suitable for CRISPR-mediated Oligonucleotide binding and/or editing comprising a locked hairpin structure comprising the nucleotide sequence SEQ ID NO. 1:
5'-X₁-XₙGGAC(N)ᵣGUCCXₙ₊₁-X₂ₙ-3'
wherein X is in each case independently any nucleotide e.g. selected from A, C, G, or U, with the proviso that X₁-Xₙ form a contiguous hairpin stem with Xₙ₊₁-X₂ₙ and n is 2, 3, 4, 5, or 6, particularly 3, 4 or 5, and more particularly 4; and
Nᵣ is the loop of the locked hairpin; and N is any nucleotide e.g. selected from A, C, G, or U, and r is 3, 4, 5 or 6, particularly 4 or 5, and more particularly 4.

A further aspect of the present invention relates to a gRNA suitable for CRISPR-mediated oligonucleotide binding and/or editing comprising a locked hairpin secondary structure comprising the nucleotide sequence SEQ ID NO. 2:
5'-ACUUGGAC(N)rGUCCAAGU-3'
wherein Nᵣ is the loop of the locked hairpin, N is any nucleotide particularly selected from A, C, G, or U, and r is 3, 4, 5 or 6, particularly 4 or 5, and more particularly 4.

A further aspect of the present invention relates to a gRNA suitable for CRISPR-mediated oligonucleotide binding and/or editing comprising a locked hairpin secondary structure comprising the nucleotide sequence SEQ ID NO. 3:
5'-ACUUGGACUUCGGUCCAAGU-3'.

A further aspect of the present invention relates to a gRNA suitable for CRISPR-mediated Oligonucleotide binding and/or editing, which comprises the nucleotide sequence SEQ ID NO. 4: 
5'AGCAUAGCAAGUUAAAAUAAGGCUAGUCCGUUAUCAACUUGGACUUCGGUC
CAAGUGGCACCGAGUCGGUGCUUU-3'
 wherein A, C, G, and U are nucleotide building blocks including modified nucleotide building blocks.

A further aspect of the present invention relates to a gRNA suitable for CRISPR-mediated Oligonucleotide binding and/or editing, which comprises the nucleotide sequence SEQ ID NO. 5: 
5'mA*mG*mCmAmUmAmGmCmAmAGUUmAAmAAUAmAmGGCUAGUCmCGUUA
mUmCAAmCmUmUGGACUUCGGUCCmAmAmGmUmGGmCmAmCmCmGmAmG
mUmCmGmGmUmGmCmU*mU*mU-3'
 wherein A, C, G, and U are non-modified nucleotide building blocks, mA, mC, mG, and mU are sugar-modified nucleotide building blocks, particularly 2'-O-methyl-modified building blocks and * denotes a modified internucleosidic linkage, particularly a phosphorothioate linkage.

A further aspect of the present invention relates to a trans-activating CRISPR RNA (tracrRNA) suitable for CRISPR-mediated Oligonucleotide binding and/or editing comprising a constant gRNA sequence comprising at least one locked hairpin secondary structure at a position that does not interfere with Oligonucleotide binding and/or editing.

A further aspect of the present invention relates to a trans-activating CRISPR RNA (tracrRNA) suitable for CRISPR-mediated Oligonucleotide binding and/or editing comprising:
- a constant gRNA sequence comprising a CRISPR repeat/anti-repeat-structure or a portion thereof and at least one locked hairpin secondary structure at a position that does not interfere with Oligonucleotide binding and/or editing.

A further aspect of the present invention relates to a trans-activating CRISPR RNA (tracrRNA) suitable for CRISPR-mediated Oligonucleotide binding and/or editing comprising:
- a constant gRNA sequence comprising in 5' to 3' direction a (i) an CRISPR anti-repeat sequence, (ii) optionally a nexus sequence and (iii) at least one locked hairpin secondary structure.

A further aspect of the present invention relates to a nucleic acid molecule encoding a gRNA or a tracrRNA as described above optionally in operative linkage with an expression control sequence.

A further aspect of the present invention relates to a library comprising a plurality of gRNAs as described above comprising different target-specific spacer sequences and a constant gRNA sequence, particularly a common constant gRNA sequence comprising at least one locked hairpin secondary structure at a position that does not interfere with Oligonucleotide binding and/or editing.

A further aspect of the present invention relates to a complex comprising a Cas enzyme and a gRNA or a tracrRNA as indicated above.

A further aspect of the present invention relates to an *in vitro* or ex *vivo* use of a gRNA, a tracrRNA, a gRNA- or tracrRNA-encoding nucleic acid, a library or a complex as described above for CRISPR-mediated Oligonucleotide binding and/or editing, particularly for genome editing in a eukaryotic cell.

A further aspect of the present invention relates to a preparation comprising a gRNA, a tracrRNA, a gRNA- or tracrRNA-encoding nucleic acid, a library or a complex as described above for use in medicine including human medicine and veterinary medicine.

A further aspect of the present invention relates to a use of a gRNA, a tracrRNA, a gRNA- or tracrRNA-encoding nucleic acid, a library or a complex as described above as described above for genome editing in a plant cell or plant organism.

### Detailed description

The present inventors have provided means for increasing the efficacy of CRISPR-mediated Oligonucleotide binding and/or editing including genome editing by designing a gRNA capable of targeting substantially any DNA sequence including sequences that have previously been refractory to CRISPR-mediated genome editing. In particular, the inventors have engineered a trans-activating crRNA (tracrRNA) with an unusually stable hairpin lock and optionally further enhanced chemically modifications. The engineered genome editing optimized locked design (GOLD)-tracR increases genome editing efficiency in a multitude of tested targets and can efficiently edit targets, which were refractory to standard CRISPR-Cas9 cleavage. The GOLD-tracR can increase genome editing up to 28-fold (from 3.2% to 89%), while the mean increase across tested targets is 6-fold. This improved gRNA will allow efficient editing regardless of spacer sequence composition and will be especially useful if a desired DNA site is difficult to edit.

The gRNA of the invention may be combined with other means of improving CRISPR-mediated Oligonucleotide binding and/or editing including the use of a gRNA comprising at least one modified, e.g. chemically stabilized nucleotide building block, a Hybridization extended A-T inversion (HEAT) of the repeat/anti-repeat-structure, non-homologous oligonucleotide enhancement (NOE), and/or use of the further enhancers of CRISPR-mediated Oligonucleotide binding and/or editing,

According to the present invention, a gRNA suitable for CRISPR-mediated Oligonucleotide binding and/or editing is provided. The gRNA comprises a locked hairpin secondary structure that does not interfere with Oligonucleotide binding and/or editing. The locked hairpin secondary structure is present in at least one position of the constant gRNA part other than a CRISPR repeat/anti-repeat duplex.

The gRNA of the invention is particularly suitable for CRISPR/Cas-mediated Oligonucleotide binding and/or editing, wherein Oligonucleotide binding and/or editing is mediated by a complex comprising a gRNA of the invention and a Cas enzyme, which may be selected from any suitable Cas enzyme, e.g. as described by Makarova et al. (2020), the content of which is herein incorporated by reference.

In certain embodiments, the Cas enzyme is selected from Cas9, Cas12a, Cas12e, Cas12b, Cas12i, Cas12h, Cas12c, Cas12d, Cas12f, Cas12g, Cas12k, Cas12j, Cas13a, Cas13b, Cas13c, Cas13d, and Cas14 including any recombinant variant thereof and particularly from Cas9 including any recombinant variant thereof. The Cas9 enzyme may be a *Streptococcus,* e.g. a S. *pyogenes* or *Lactobacillus* Cas9 enzyme as described by Briner et al. (2014), the content of which is herein incorporated by reference, including any recombinant variant thereof. In particular, the term "Cas enzyme" includes optimized Cas enzymes, e.g. Cas enzymes resulting in less off-target events such as R691A Cas9-Hifi and/or having a higher catalytic efficacy such as K918N SpyCECas9. Further, the term "Cas enzyme" as used herein includes a catalytically fully active Cas nuclease capable of creating a double-strand break in a DNA template, a catalytically partially active Cas nickase capable of creating a single-strand break in a double stranded DNA template, e.g. Cas9 D10A or Cas9 H840A, and a catalytically inactive Cas enzyme, e.g. dCas9. Further, the term "Cas enzyme" includes split-fusion versions of the above enzymes, e.g. split-fusion versions of Cas9 or Cas9 D10A.

The term "CRISPR-mediated Oligonucleotide binding and/or editing" includes binding and optionally cleaving of any DNA or RNA template, particularly a double-stranded DNA template or a single-stranded DNA template or a single-stranded RNA template, e.g. an oligonucleotide, a plasmid, a chromosome *in vitro* and *in vivo.* For example, the term includes activation and/or repression, e.g. dCas9 gene activation and/or repression, base editing, genome editing, epigenome editing and/or prime editing.

The gRNA of the invention comprises a plurality of modules depending from the structural requirements of the enzyme, e.g. the Cas enzyme, with which it forms a ribonucleoprotein complex that is capable of Oligonucleotide binding and/or editing.

A gRNA of the invention comprises a target-specific spacer sequence, i.e. a sequence capable of specifically binding to a given target oligonucleotide sequence. In certain embodiments, the target-specific spacer sequence is positioned at the 5'-end of the gRNA. In certain embodiments, the target-specific spacer is positioned at the 3'-end of the gRNA. Typically, the target-specific spacer has a length of about 12-30 nt, particularly 15-27 nt and more particularly about 17-24 nt.

A gRNA of the invention further comprises constant gRNA sequence, i.e. a sequence portion, which is independent from a given target oligonucleotide sequence. The constant gRNA sequence of the invention comprises at least one locked hairpin secondary structure at a position that does not interfere with Oligonucleotide binding and/or editing.

The term "locked hairpin secondary structure" in any gRNA of the invention means that the hairpin has a high stability, particularly a high thermostability.

In certain embodiments, the locked hairpin of a gRNA of the present invention has a secondary structure comprising a contiguous stem. The term "contiguous stem" means an uninterrupted sequence of base pairs in the stem, i.e. without any bulge formed of unpaired nucleotides. In certain embodiments, the locked hairpin of a gRNA of the present invention has a secondary structure comprising a contiguous stem having a length of at least about 5 nt, e.g. about 6 nt to about 12 nt. In particular embodiments, the locked hairpin has a secondary structure comprising a contiguous stem having a length of 6, 7, 8, 9 or 10 nt, more particularly of 8 nt. The "term" length in "nt" corresponds to the number of contiguous base pairs of the stem.

In certain embodiments, the locked hairpin of a gRNA of the present invention has a secondary structure comprising a contiguous stem comprising at least 2 C-G base pairs, e.g. about 3 C-G base pairs to about 6 C-G base pairs. In particular embodiments, the locked hairpin forms a secondary structure comprising a contiguous stem comprising 2, 3, 4 or 5 C-G base pairs, more particularly 4 C-G base pairs. In this context, the term "C-G base pair" encompasses any base pair formed by a G nucleotide and a C nucleotide irrespectively from their orientation within the stem.

In certain embodiments, the locked hairpin of a gRNA of the present invention forms a secondary structure comprising a contiguous stem having a melting temperature of at least about 60°C, e.g. at least about 65°C or at least about 70°C and up to about 90°C as determined by e.g. differential scanning calorimetry and UV absorbance spectroscopy.

Further, the term "locked hairpin secondary structure" in any gRNA of invention means that the hairpin has a high stability, particularly a high thermostability e.g. compared to an unlocked hairpin of a standard gRNA.

In this context, it is to be understood that an unlocked hairpin of a standard CRISPR/Cas9-gRNA, forms a stem-loop structure having a contiguous stem with a length of 4 nt and a loop of 4 nt. More particularly, a first hairpin of a standard gRNA as shown in Fig. 1A has the nucleotide sequence of SEQ ID NO. 6:
5'-ACUUGAAAAAGU-3'

The term "locked secondary structure" further means that the secondary structure of the first hairpin comprises a contiguous stem of base-paired nucleotide building blocks and particularly a contiguous stem of base-paired nucleotide building blocks structure connected by a loop of unpaired nucleotide building blocks. The term "contiguous stem" excludes the presence of a bulge with a directional kink with the stem. The term "base-paired" refers to base pairs formed by a purine nucleobase such as A or G to a pyrimidine nucleobase such as U or C. In particular, the term "base pair" refers to the base pairs A-U, U-A, G-C and C-G, but also includes G-U, and A-C.

The locked hairpin secondary structure does not interfere with oligonucleotide binding and/or editing. In certain embodiments, the locked hairpin secondary structure does not disrupt interactions of gRNA bases with amino acids, e.g. interactions selected from hydrogen-bonding, formation of salt bridges, and/or stacking/hydrophobic interactions of an oligonucleotide binding and/or editing enzyme, e.g. Cas enzyme, which are necessary for oligonucleotide binding and/or editing. A potential disruptive effect of a gRNA modification may be determined by a crystal structure analysis of a gRNA-enzyme complex whereby amino acids interacting with gRNA bases can be identified and optionally confirmed by an oligonucleotide binding and/or editing assay comparing wild-type gRNA without a locked hairpin secondary structure and modified gRNA with a locked hairpin secondary structure, in particular by gel-electrophoresis of an enzyme complexed with gRNA and target oligonucleotide.

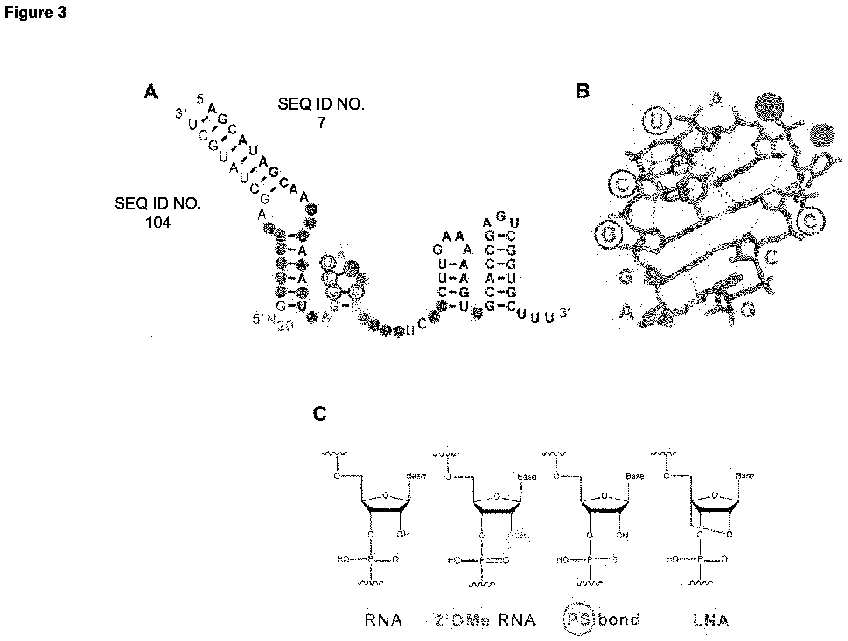

In certain embodiments, the locked hairpin secondary structure does not comprise a base pair that interacts with the Oligonucleotide binding and/or editing enzyme, e.g. the Cas enzyme, wherein the locked hairpin secondary structure does not comprise a base pair where the 2'OH groups of both nucleotides forming the base pair interact with an Oligonucleotide binding and/or editing enzyme (cf. Fig. 3A)..

Further, the locked hairpin secondary structure of the invention is different from a CRISPR repeat/anti-repeat structure. In certain embodiments, a repeat/anti-repeat structure of a gRNA comprises a lower stem, optionally a bulge with a directional kink and optionally an upper stem. Further, a repeat/anti-repeat structure of a gRNA comprises at least one base pair, e.g. 2 or 3 base pairs that interact with an Oligonucleotide binding and/or editing enzyme wherein the repeat/anti-repeat structure comprises at least one base pair, e.g. 2 or 3 base pairs where the 2'OH groups of both nucleotides forming the base pair interact with an Oligonucleotide binding and/or editing enzyme (c.f. Fig. 3A). In contrast, a locked hairpin secondary structure as defined herein typically has an uninterrupted stem. Further, it does not interact an Oligonucleotide binding and/or editing Cas enzyme.

In certain embodiments, the gRNA of the invention consists of a single RNA molecule, e.g. a single guide RNA (sgRNA) molecule. In further embodiments, the gRNA of the invention comprises at least two separate RNA molecules, e.g. a spacer-containing CRISPR RNA (crRNA) molecule and a trans-activating CRISPR RNA (tracrRNA) molecule. In still further embodiments, the gRNA of the invention comprises three or more separate RNA molecules, e.g. a spacer-containing CRISPR RNA (crRNA) molecule, and a trans-activating CRISPR RNA (tracrRNA) molecule comprised of two separate RNA molecules.

In certain embodiments, the gRNA comprises
- a target-specific spacer sequence and
- a constant gRNA sequence comprising (i) a CRISPR repeat/anti-repeat-structure that interacts with an Oligonucleotide binding and/or editing enzyme, e.g. a Cas enzyme, and (ii) at least one locked hairpin secondary structure at a
position that does not interfere with Oligonucleotide binding and/or editing and particularly wherein the locked hairpin secondary structure does not interact with said Oligonucleotide binding and/or editing enzyme.

In certain embodiments, a gRNA of the invention is suitable for CRISPR/Cas-mediated Oligonucleotide binding and/or editing wherein the Oligonucleotide binding and/or editing enzyme is selected from one of the Cas enzymes as listed below. A gRNA adapted for such an enzyme may comprise the following structural modules in the 5'-3' direction:

| | |
|---|---|
| Cas9 | spacer - CRISPR repeat/anti-repeat- hairpin(s) |
| Cas12a | CRISPR repeat/hairpin - spacer |
| Cas12b | hairpin(s) - CRISPR repeat/anti-repeat - spacer |
| Cas12c | hairpin(s) - CRISPR repeat/anti-repeat - spacer |
| Cas12e | hairpin(s) - CRISPR repeat/anti-repeat - spacer |
| Cas12f | hairpin(s) - CRISPR repeat/anti-repeat - spacer |
| Cas12g | hairpin(s) - CRISPR repeat/anti-repeat - spacer |
| Cas12h | CRISPR repeat/hairpin - spacer |
| Cas12i | CRISPR repeat/hairpin - spacer |
| Cas12j | CRISPR repeat/hairpin - spacer |
| Cas12k | hairpin(s) - CRISPR repeat/anti-repeat - spacer |
| Cas13a | spacer- CRISPR repeat/hairpin |
| Cas13b | spacer- CRISPR repeat/hairpin |
| Cas13c | spacer - CRISPR repeat/hairpin |
| Cas13d | spacer - CRISPR repeat/hairpin |
| Cas14 | hairpin(s) - CRISPR repeat/anti-repeat - spacer |

The term "CRISPR repeat/hairpin" as used above relates to a CRISPR hairpin structure present in a single RNA molecule. The term "CRISPR repeat/anti-repeat" as used above refers to a CRISPR hairpin structure present in two separate RNA molecules forming the gRNA, e.g. a crRNA molecule and a tracrRNA molecule. The term "hairpin(s)" as used above refers to a non-CRISPR repeat or repeat/anti-repeat structure.

A hairpin having a locked secondary structure according to the present invention may be an extension of an already present non-CRISPR hairpin or a newly introduced hairpin, e.g. 5' or 3' to a "CRISPR repeat/hairpin" or "CRISPR repeat/anti-repeat" structure.

In particular embodiments, the gRNA is suitable for CRISPR/Cas9-mediated Oligonucleotide binding and/or editing and comprises in 5' to 3' direction:
- a target-specific spacer sequence and
- a constant gRNA sequence comprising in 5' to 3' direction a (i) repeat/anti-repeat structure, (ii) optionally a nexus sequence and (iii) at least one locked hairpin secondary structure.

Preferred structural elements of a gRNA of the invention are outlined in the following in detail in in particular reference to a gRNA of the invention suitable for CRISPR/Cas9-mediated Oligonucleotide binding and/or editing. It should be noted, however, that certain of the described structural elements may also be present in other types of gRNAs.

A gRNA of the invention suitable for CRISPR/Cas9-mediated Oligonucleotide binding and/or editing may comprise in 5' to 3' direction: (i) a target-specific spacer sequence, (ii) a repeat/anti-repeat structure comprising a lower stem, optionally a bulge with a directional kink and optionally an upper stem, (iii) optionally a nexus sequence and (iv) a hairpin sequence comprising at least one locked hairpin secondary structure. In certain embodiments, the hairpin sequence (iv) of the gRNA may include in 5' to 3' direction a first hairpin and optionally a second hairpin wherein at least the first hairpin has a locked secondary structure.

In particular embodiments, the gRNA of the invention comprises in 5' to 3' direction (i) a target-specific spacer sequence, (ii) a repeat/anti-repeat structure comprising a lower stem, a bulge with a directional kink and an upper stem, (iii) a nexus sequence and (iv) a hairpin sequence comprising in 5' to 3' direction a first hairpin and a second hairpin wherein at least the first hairpin has a locked secondary structure.

In certain embodiments, the gRNA of the invention consists of a single RNA molecule. For example, the gRNA may be a single guide RNA (sgRNA) molecule wherein the repeat/anti-repeat structure comprises a non-contiguous hairpin structure comprising a lower stem, a bulge with a directional kink, an upper stem and a fusion loop.

In further embodiments, the gRNA of the invention comprises at least two separate RNA molecules. For example, the gRNA consists of two separate RNA molecules, e.g. a first RNA molecule comprising in 5' to 3' direction a target-specific spacer sequence and a repeat portion of a repeat/anti-repeat structure and a second RNA molecule comprising in 5' to 3' direction an anti-repeat portion of a repeat/anti-repeat structure, optionally a nexus sequence, and a hairpin sequence comprising at least one locked hairpin secondary structure, e.g. a first hairpin and optionally a second hairpin wherein at least the first hairpin has a locked secondary structure. More specifically, the first RNA molecule is a spacer-containing CRISPR RNA (crRNA) molecule and the second RNA molecule is a trans-activating CRISPR RNA (tracrRNA) molecule.

In still further embodiments, the gRNA of the invention comprises three or more separate RNA molecules. For example, the gRNA may comprise three RNA molecules, e.g. a spacer-containing CRISPR RNA (crRNA) molecule, and a trans-activating CRISPR RNA (tracrRNA) molecule comprised of two separate RNA molecules separated at the stem region of a hairpin within the hairpin sequence, e.g. in the stem region of a hairpin, e.g. in the stem region of a hairpin having a locked secondary structure. In such an embodiment, the gRNA comprises a crRNA molecule and a tracrRNA comprising at least two separate RNA molecules including a tracrRNA-A molecule and a tracrRNA-B molecule. The tracrRNA-A molecule comprises an anti-repeat sequence, optionally a nexus sequence and a first 5'-portion of the hairpin sequence and the tracrRNA-B molecule comprises a second 3'-portion of the hairpin sequence. The 3'-end of the tracrRNA-A molecule and the 5'-end of the tracrRNA-B molecule form a hairpin, particularly a hairpin, which forms a locked secondary structure comprising a contiguous stem as herein described *infra* without a loop.

A gRNA suitable for Cas9-mediated Oligonucleotide binding and/or editing comprises at its 5'-end a target-specific spacer sequence, i.e. a sequence capable of binding to a target oligonucleotide sequence. Typically, the target-specific spacer sequence has a length of about 15-27 nt, particularly about 17-24 nt. An example of a spacer sequence specific for the SSH2 gene is shown in Fig. 1A, i.e. the 5'-part of the crRNA.

3' to the target-specific spacer sequence, the gRNA comprises a repeat sequence, i.e. a sequence, which is capable of forming a secondary structure with an anti-repeat sequence. The repeat/anti-repeat secondary structure comprises a lower stem, optionally a bulge with a directional kink, and optionally an upper stem. The lower stem and the upper stem are formed by base-paired nucleotide building blocks from the repeat sequence and the anti-repeat sequence respectively interrupted by a bulge consisting of one or more unpaired building blocks. The bulge comprises a directional kink, i.e. the stretches of unpaired nucleotide building blocks in the repeat sequence and in the anti-repeat sequence have different lengths. In certain embodiments, the upper stem may be removed and the bulge replaced by a loop as described by Briner et al. (2014) the content of which is herein incorporated by reference.

The repeat sequence typically has a length of about 8 to about 30 nt including the lower stem, and - if present - the bulge and the upper stem sequences. For example, the sequence forming the lower stem has a length of about 5 to about 7 nt, the sequence forming the bulge has a length of about 1 to about 5 nt, e.g. 2 nt and the sequence forming the upper stem has a length of about 4 to about 20 nt. In certain embodiments, the sequence forming the upper stem is elongated compared to a standard gRNA and has a length of about 7 to about 20 nt, e.g. 8 nt as described by Chen et al., 2013. A preferred example of a repeat sequence having an elongated upper stem is shown in Fig. 1A, i.e. the 3'-part of the crRNA.

As outlined above, a gRNA of the invention may consist of a single RNA molecule, e.g. a sgRNA. In such an embodiment, a repeat/anti-repeat secondary structure formed between the repeat sequence and the anti-repeat sequence may comprise a lower stem, optionally a bulge with a directional kink, and optionally an upper stem and additionally a loop, which connects the 3'-end of the repeat and the 5'-end of the anti-repeat. The loop of the repeat/anti-repeat secondary structure comprises a sequence of unpaired nucleotide building blocks, e.g. consisting of about 3 to about 10 nt and particularly of 4 nt.

Alternatively, the gRNA may be comprised of two or more separate RNA molecules. In such an embodiment, the first RNA molecule may comprise the target-specific spacer and the repeat sequence having a free 3'-end after the repeat sequence, particularly after the upper stem sequence as shown in Fig. 1A. In such an embodiment, the repeat sequence and the anti-repeat sequence form an interrupted repeat/anti-repeat secondary structure without being connected via a loop.

3' to the repeat sequence, the gRNA comprises an anti-repeat sequence i.e. a sequence, which is capable of forming a repeat/anti-repeat secondary structure with the repeat sequence.

Typically, the anti-repeat sequence has a different length compared to the repeat sequence resulting in a bulge with a directional kink. In certain embodiments, the anti-repeat sequence is longer, e.g. about 2 nucleotides longer than the repeat sequence. Alternatively, the anti-repeat-sequence may be shorter, e.g. about 2 nt shorter than the repeat sequence as described by Briner et al. (2014). Typically, the anti-repeat sequence has a length of about 10 to about 32 nt including the lower stem, and - if present - the bulge and the upper stem sequences. For example, the sequence forming the lower stem has a length of about 5 to about 7 nt, the sequence forming the bulge has a length of about 1 to about 5 nt, e.g. 4 nt, and the sequence forming the upper stem has a length of about 4 to about 20 nt. In certain embodiments, the sequence forming the upper stem is elongated compared to a standard gRNA and has a length of about 7 to about 20 nt, e.g. 8 nt, as described by Chen et al., 2013. A preferred example of an anti-repeat sequence having an elongated upper stem is shown in Fig. 1A, i.e. the 5'-part of the tracR.

As outlined above, the 5'-end of the anti-repeat sequence may be covalently linked to the 3'-end of the repeat sequence via a loop as described above. Alternatively, there is a free 5'-end at the begin of the anti-repeat sequence, particularly at the begin of the upper stem sequence as shown in Fig. 1A.

3' to the anti-repeat sequence, the gRNA optionally comprises a nexus sequence, i.e. a sequence capable of forming a secondary structure and interacting with a Cas enzyme. Typically, the nexus sequence comprises two conserved A residues followed by a stem-loop structure having a bulge, particularly a single unpaired U in the 3'-stem sequence, particularly a nexus sequence as shown in Fig. 1A. The length of the nexus sequence is typically about 9 to about 12 nt.

3' to the anti-repeat sequence or, if present to the nexus sequence, a CRISPR/Cas9 gRNA comprises a hairpin sequence comprising at least one hairpin. Preferably, the hairpin sequence comprises a first hairpin, which is the first, i.e. most 5'-positioned hairpin after the repeat/anti-repeat structure or - if present - the nexus sequence. The first hairpin is a sequence, which forms a non-interrupted stem-loop structure. In a standard gRNA, the first hairpin typically forms a contiguous secondary structure of paired nucleotide building blocks, i.e. a stem, with a length of 4 nt and a loop consisting of 4 nt. An example of a first hairpin is shown in Fig. 1A.

3' to the first hairpin sequence, the hairpin structure may comprise a second hairpin sequence, i.e. a sequence, which is positioned 3'- to the first hairpin and forms a further non-interrupted stem-loop structure. In a standard gRNA, the second hairpin typically forms typically forms a stem with a stem length of 6 nt and a loop consisting of 3 nt. An example of a second hairpin is shown in Fig. 1A.

At its 3'-end, a CRISPR/Cas9-gRNA typically comprises a stretch of U nucleotide building blocks, e.g. about 2 to about 10 U building blocks.

Optionally, a gRNA of the invention may comprise additional sequence elements, e.g. a 3'- prime binding sequence for prime editing, e.g. as described by Bhagwat et. (2020) the content of which is herein incorporated by reference.

According to the present invention, the nucleotide sequence of a hairpin is altered compared to a standard gRNA and forms a locked secondary structure.

A CRISPR/Cas9-gRNA typically comprises a hairpin sequence comprising a first hairpin and a second hairpin. The term "first hairpin" relates to the most 5'-positioned hairpin in the hairpin sequence. The hairpin sequence including the first hairpin does not substantially interact with a Cas enzyme, particularly a Cas9 enzyme. In particular embodiments, the hairpin having a locking secondary structure does not comprise a base pair where the 2'OH groups of both nucleotides forming the base pair interact with an Oligonucleotide binding and/or editing enzyme.

The hairpin sequence including the first hairpin is distinct from the repeat/anti-repeat secondary structure of the gRNA, which - as outlined above - typically comprises a lower stem, a bulge with a directional kink and an upper stem. Further, the hairpin sequence including the first hairpin is distinct from the nexus as outlined above. The hairpin sequence including the first hairpin is positioned 3' to the repeat/anti-repeat secondary structure and to the nexus, if present.

In particular embodiments, the locked hairpin of a gRNA of the present invention comprises the nucleotide sequence SEQ ID NO. 1:
5'-X₁-XₙGGAC(N)ᵣGUCCXₙ₊₁-X₂ₙ-3'
wherein X is any nucleotide e.g. selected from A, C, G, or U, with the proviso that X₁-Xₙ form a contiguous hairpin stem with Xₙ₊₁-X₂ₙ and n is 2, 3, 4, 5, or 6, particularly 3, 4 or 5, and more particularly 4;
wherein Nᵣ is the loop of the locked hairpin; and N is any nucleotide e.g. selected from A, C, G, or U, and r is 3, 4, 5 or 6, particularly 4 or 5, and more particularly 4.

The term "A" as used herein particularly denotes a nucleotide building block comprising an adenine nucleobase, a ribose a phosphate group or internucleosidic linkage. In some embodiments, the term specifies an unmodified nucleotide building block. In some embodiments, the term also includes a modified nucleotide building block as described herein *infra.*

The term "G" as used herein particularly denotes a nucleotide building block comprising a guanine nucleobase, a ribose a phosphate group or internucleosidic linkage. In some embodiments, the term specifies an unmodified nucleotide building block. In some embodiments, the term also includes a modified nucleotide building block as described herein *infra.*

The term "C" as used herein particularly denotes a nucleotide building block comprising a cytosine nucleobase, a ribose a phosphate group or internucleosidic linkage. In some embodiments, the term specifies an unmodified nucleotide building block. In some embodiments, the term also includes a modified nucleotide building block as described herein *infra.*

The term "U" as used herein particularly denotes a nucleotide building block comprising an uracil nucleobase, a ribose a phosphate group or internucleosidic linkage. In some embodiments, the term specifies an unmodified nucleotide building block. In some embodiments, the term also includes a modified nucleotide building block, e.g. a nucleotide building block including a thymine nucleobase as described herein *infra.*

It is noted, that at certain positions of a gRNA, deoxyribonucleotide building blocks may be present, as described by Rueda et al. (2017), the content of which is herein incorporated by reference.

In further particular embodiments, the locked hairpin comprises the nucleotide sequence SEQ ID NO. 2:
5'-ACUUGGAC(N)rGUCCAAGU-3'. wherein Nᵣ is the loop of the locked hairpin, N is any nucleotide particularly selected from A, C, G, or U, and r is 3, 4, 5 or 6, particularly 4 or 5, and more particularly 4.

In further particular embodiments, the locked hairpin comprises a loop having a nucleotide sequence selected from:
(i) 5'-GNRA-3'
(ii) 5'-UNCG-3' and
(iii) 5'- CUUG-3',
wherein R is a nucleotide selected from A or G and N is any nucleotide e.g. selected from A, C, G, or U.

In further particular embodiments, the locked hairpin comprises a loop having a nucleotide sequence selected from:
(i) 5'-UUCG-3' and
(ii) 5'-CUUG-3'.

In further particular embodiments, the locked hairpin comprises the nucleotide sequence SEQ ID NO. 3:
5'-ACUUGGACUUCGGUCCAAGU-3'

In further particular embodiments, the gRNA or tracrRNA of the invention comprises the nucleotide sequence SEQ ID NO. 4: 
5’-
AGCAUAGCAAGUUAAAAUAAGGCUAGUCCGUUAUCAACUUGGACUUCGGUCC
AAGUGGCACCGAGUCGGUGCUUU-3’
 wherein A, C, G, and U are nucleotide building blocks including modified nucleotide building blocks as described *infra.*

In certain embodiments, the gRNA comprises at least one modified nucleotide building block. In alternative embodiments, the gRNA does not comprise any modified nucleotide building block.

The term "modified nucleotide building block" relates to a nucleotide building block, which differs from a naturally occurring RNA building block. In particular, the term includes (a) a nucleobase-modified building block, (b) a sugar-modified building block, (c) a backbone-modified building block, (d) a modified nucleotide building block having attached thereto a heterologous moiety, and (e) any combination thereof.

A nucleobase-modified building block comprises a non-RNA nucleobase, i.e. a nucleobase different from a naturally occurring RNA nucleobase, i.e. adenine, guanine, cytosine and uracil. For example, a non-RNA nucleobase is thymine.

A sugar-modified building block comprises a non-ribose sugar moiety, i.e. sugar moiety different from ribose. For example, sugar-modified building block may be selected from:
(i) a sugar-modified building block comprising a modified substituent at the 2'-position of the ribose moiety, particularly a substituent 2'-R wherein R is H, halo, e.g. F, Cl, Br, or I, C₁-C₅ alkyl, O-C₁-C₅ alkyl, S-C₁-C₅ alkyl, C₂-C₅ alkenyl, O-C₂-C₅ alkenyl, S-C₂-C₅ alkenyl, C₂-C₅ alkynyl, O-C₂-C₅ alkynyl, S-C₂-C₅ alkynyl or amino including mono- and disubstituted amino,
(ii) a sugar-modified building block, wherein two non-neighboring ring atoms of the ribose moiety are connected by a bridge, particularly a 2'-4'-bridged building block wherein the bridge typically has a length of 2-5 atoms, particularly 2 or 3 atoms including C-atoms and optionally heteroatoms such as O, N or S, or
(iii) a sugar-modified building block wherein the ring of the ribose moiety is modified, e.g. a morpholino moiety, a thio-ribose moiety, or a 6-membered pyranose moiety.

In specific embodiments, the sugar-modified nucleotide building block may be a 2'-O Methyl ribose wherein the substituent at the 2'-position of the ribose moiety is 2'-OCH₃. In further specific embodiments, the sugar-modified nucleotide building block may be a locked nucleic acid (LNA) building block having a 2'-O-CH₂-4' bridge.

A backbone-modified building block may comprise a modified internucleosidic linkage, i.e. an internucleosidic linkage different from a phosphate group. For example, the backbone-modified building block may comprise a phosphorothioate linkage.

In still further embodiments, the modified nucleotide building block may have attached thereto, e.g. attached to the nucleobase, a heterologous moiety, e.g. a lipid moiety or a polyethylene glycol moiety.

In particular embodiments, the gRNA may comprise a plurality of modified nucleotide building blocks, e.g. at least about 10, at least about 15, at least about 20, at least about 30 or more modified nucleotide building blocks.

Further, modified building blocks are typically present at positions, where they not negatively interact with a Cas enzyme, i.e. where they do not or do not substantially inhibit Cas activity. In certain embodiments, at least one modified nucleotide building block, e.g. a sugar-modified building block is present in a portion of the gRNA selected from the repeat sequence, e.g. the 5'-part thereof, the anti-repeat-sequence, e.g. the 3'-part thereof, and the hairpin structure, e.g. the first hairpin and, if present, the second hairpin. Further, at least one backbone-modified building block may be present at a 5'-end and/or at a 3'-end of the gRNA, and particularly at least two backbone-modified building blocks may be present at a 5'-end and/or at a 3'-end of the gRNA. In further embodiments, wherein the nexus loop sequence does not comprise a modified nucleotide building block.

In particular embodiments, the locked hairpin comprises at least one modified nucleotide building block, e.g. at least one sugar-modified nucleotide building block as described above.

For example, the locked hairpin comprises at least one modified nucleotide building block, particularly at least one sugar-modified building block in one or more or all of the nucleotides X₁-Xₙ and Xₙ₊₁-X₂ₙ according to SEQ ID NO: 1 and/or the locked hairpin does not comprise a modified nucleotide building block in one or more or all of the nucleotides GGAC(N)ᵣGUCC according to SEQ ID NO: 1.

In particular embodiments, the gRNA comprises the nucleotide sequence SEQ ID NO. 5: 
5’-
mA*mG*mCmAmUmAmGmCmAmAGUUmAAmAAUAmAmGGCUAGUCmCGUUAm
UmCAAmCmUmUGGACUUCGGUCCmAmAmGmUmGGmCmAmCmCmGmAmGm
UmCmGmGmUmGmCmU*mU*mU-3’
 wherein A, C, G, and U are non-modified nucleotide building blocks, mA, mC, mG, and mU are sugar-modified building blocks, particularly 2'-O-Methyl-modified building blocks and * denotes a modified internucleosidic linkage, particularly a phosphorothioate linkage.

As outlined above, the gRNA of the invention may be a single RNA molecule, i.e. a sgRNA or comprise at least two separate RNA molecules, e.g. a crRNA and a tracrRNA.

Thus, a further aspect of the present is a tracrRNA suitable for CRISPR-mediated Oligonucleotide binding and/or editing comprising a constant gRNA sequence comprising at least one hairpin having a locked secondary structure that does not interfere with Oligonucleotide binding and/or editing and wherein the locked hairpin secondary structure does comprise a base pair that interacts with an Oligonucleotide binding and/or editing enzyme.

The tracrRNA of the invention is capable of forming a gRNA with a separate crRNA. Further, the tracrRNA may comprise eat least one of the features as described above for the gRNA.

In particular embodiments, the tracrRNA consists of the nucleotide sequence SEQ ID NO. 5: 
5’-
mA*mG*mCmAmUmAmGmCmAmAGUUmAAmAAUAmAmGGCUAGUCmCGUUAm
UmCAAmCmUmUGGACUUCGGUCCmAmAmGmUmGGmCmAmCmCmGmAmGm
UmCmGmGmUmGmCmU*mU*mU-3’
 wherein A, C, G, and U are non-modified nucleotide building blocks, mA, mC, mG, and mU are sugar-modified building blocks, particularly 2'-O-Methyl-modified building blocks and * denotes a modified internucleosidic linkage, particularly a phosphorothioate linkage.

A tracrRNA molecule of SEQ ID NO. 5 comprising phosphorothioate bonds at positions:1, 2, 73, and 74 and 2'OMe modifications at positions 1-10, 14, 16, 20, 21, 29, 34, 35, 38-40, 53-56, 58-75 is designated as GOLD-tracR in the present Examples.

The gRNA or tracrRNA of the present invention may be prepared by known methods including chemical synthesis, or transcription *in vitro* or *in vivo.* Chemical synthesis, e.g. solid phase synthesis, is particularly suitable for the preparation of RNA molecules comprising at least one modified nucleotide building block. Alternatively, the gRNA or tracrRNA of the invention may be prepared by transcription from a nucleic acid template molecule, e.g. from a DNA template comprising an expression control sequence in operative linkage with a sequence encoding the gRNA or tracrRNA. In *vitro* transcription may be performed with a suitable RNA polymerase, e.g. a bacteriophage RNA polymerase such as T7 or SP6 RNA polymerase. *In vivo* transcription may be performed by introducing a nucleic acid template, e.g. a DNA molecule encoding the gRNA or tracrRNA into a cell and expressing it therein. The nucleic acid template may be introduced as an expression vector such as a plasmid in operative linkage with appropriate expression control elements for transient or stable expression in a cell. Suitable transfection techniques for introducing proteins or nucleic acids into cells, e.g. eukaryotic cells are well known in the art and include lipofection, electroporation, e.g. nucleofection, Ca-phosphate or virus-based methods.

Still a further aspect of the present invention is a gRNA- or tracrRNA- encoding nucleic acid molecule, e.g. a DNA molecule comprising an expression control sequence, e.g. a transcriptional promoter and optionally further elements in operative linkage with a sequence encoding a gRNA or tracrRNA of the invention.

A further aspect of the present invention is a library comprising a plurality of gRNAs as described above comprising different target-specific spacer sequences and a constant gRNA sequence, particularly a common constant gRNA sequence comprising at least one locked hairpin secondary structure at a position that does not interfere with Oligonucleotide binding and/or editing. For example, the target-specific spacer sequences of individual gRNAs may be directed against different portions of a single target oligonucleotide sequence, e.g. a single gene, against different target sequences, e.g. different genes or any combination thereof. In certain embodiments, the library comprises at least two gRNAs, e.g. about 10 to about 10⁶ or even more. The library may comprise different sgRNAs comprising individual target-specific spacer sequences and common constant gRNA sequences, particularly a common locked hairpin secondary structure, optionally a common repeat/anti-repeat structure, and/or a common nexus sequences. Alternatively, the library may comprise different gRNAs composed of crRNAs comprising individual target-specific spacer sequences and a common tracrRNAs.

Still a further aspect of the invention is a complex, particularly a non-covalent ribonucleoprotein complex comprising a Cas enzyme and a gRNA as described above, or a complex, particularly a non-covalent ribonucleoprotein complex comprising a Cas enzyme and a tracrRNA as described above.

The invention further relates to the use of the gRNA, the tracrRNA, the gRNA- or tracrRNA-encoding nucleic acid molecule, the library, or the complex as described above for CRISPR-mediated Oligonucleotide binding and/or editing, particularly for CRISPR/Cas-mediated Oligonucleotide binding and/or editing *in vitro, ex vivo* or *in vivo,* e.g. in a cell-free system, in an isolated cell, in an organ or in an organism. The use includes non-medical applications, e.g. as research tool or medical applications, e.g. for *in vivo* or *ex vivo* use. In particular embodiments, a library may be used in screening applications, e.g. in unbiased screening applications, in order to identify target sequences suitable for CRISPR-mediated Oligonucleotide binding and/or editing.

In particular, the invention relates to the use of the gRNA, the tracrRNA, the gRNA- or tracrRNA-encoding nucleic acid molecule and the complex as described above for CRISPR-mediated Oligonucleotide binding and/or editing in a eukaryotic cell or a eukaryotic organism or a part, e.g. organ of a eukaryotic organism. The eukaryotic cell may be selected from an animal cell, e.g. a mammalian cell including a human cell, or a plant cell. In particular embodiments, the cell is a stem cell including a pluripotent stem cell, e.g. a human pluripotent stem cell, or an immortalized cell, e.g. a tumor cell. The eukaryotic organism may be selected from an animal, e.g. a mammal including a non-human mammal and a human being.

Still a further aspect of the present invention relates to a preparation comprising a gRNA, a tracrRNA, a gRNA- or tracrRNA-encoding nucleic acid molecule or a complex as described above for use in medicine including human medicine and veterinary medicine. Further, the preparation may comprise a pharmaceutically acceptable excipient, e.g. a polycationic compound such as a polycationic lipid. The preparation may be administered by known routes, e.g. by injection or infusion.

Still a further aspect of the present invention relates to the use of a gRNA, a tracrRNA, a gRNA- or tracrRNA-encoding nucleic acid molecule or a complex as described above for genome editing in a plant cell, plant organism or part thereof.

The gRNA, tracrRNA, gRNA- or tracrRNA-encoding nucleic acid molecule or complex as described above may be used alone or in combination with a further agent, e.g. an agent suitable for enhancing CRISPR-mediated Oligonucleotide binding and/or editing. For example, the further agent may be selected from an inhibitor of histone deacetylase (HDAC) an inhibitor of NEDD8 activating enzyme (NAE), an inhibitor of DNA-dependent Protein Kinase (DNA-PK) in particular of its catalytic subunit (DNA-PKcs), and an inhibitor of replication protein A (RPA) and any combination of compounds selected from these different classes of inhibitors. Examples of suitable inhibitors and combinations are described in WO2018/189186 the content of which is herein incorporated by reference. A particularly preferred DNA-PK inhibitor is M3814 (nedisertib) as described in WO2020/127738 the content of which is herein incorporated by reference. Additionally or alternatively, the further agent may be a mutated and catalytically inactive DNA-PK, particularly a mutated and catalytically inactive DNA-PKcs subunit as described in WO2018/189186.

### Items of the Specification

The following items describe preferred embodiments of the specification.
1) A guide RNA (gRNA) suitable for CRISPR-mediated Oligonucleotide binding and/or editing, comprising:
   - a target-specific spacer sequence and
   - a constant gRNA sequence comprising at least one locked hairpin secondary structure at a position that does not interfere with Oligonucleotide binding and/or editing.
2) A guide RNA (gRNA) suitable for CRISPR-mediated oligonucleotide binding and/or editing, particularly of item 1, comprising:
   - a target-specific spacer sequence and
   - a constant gRNA sequence comprising a CRISPR repeat/anti-repeat-structure or a portion thereof and at least one locked hairpin secondary structure at a position that does not interfere with Oligonucleotide binding and/or editing.
3) A guide RNA (gRNA) suitable for CRISPR/Cas9-mediated Oligonucleotide binding and/or editing, particularly of item 1 or 2, comprising in 5' to 3' direction:
   - a target-specific spacer sequence and
   - a constant gRNA sequence comprising in 5' to 3' direction a (i) repeat/anti-repeat structure, (ii) optionally a nexus sequence and (iii) at least one locked hairpin secondary structure.
4) The gRNA of any one of items 1-3, which is suitable for CRISPR/Cas-mediated Oligonucleotide binding and/or editing including gene activation and/or repression, base editing, genome editing, epigenome editing and/or prime editing.
5) The gRNA of any one of items 1-4, which comprises in 5'-3'-direction a target-specific spacer sequence, a repeat sequence, an anti-repeat sequence, optionally a nexus, and a hairpin sequence comprising a first hairpin and optionally a second hairpin and wherein at least the first hairpin has a locked secondary structure.
6) The gRNA of any one of items 1-5, which consists of a single RNA molecule.
7) The gRNA of item 6, which is a single guide RNA (sgRNA) molecule.
8) The gRNA of item 6 or 7, which comprises in 5'-3'direction a target-specific spacer, a repeat/anti-repeat sequence structure, optionally a nexus, and a hairpin sequence comprising a first hairpin and optionally a second hairpin and wherein at least the first hairpin has a locked secondary structure.
9) The gRNA of any one of items 1-8, which comprises at least two RNA molecules.
10) The gRNA of item 9, which consists of two RNA molecules, particularly a spacer-containing CRISPR RNA (crRNA) molecule and a trans-activating CRISPR RNA (tracrRNA) molecule.
11) The gRNA of item 9, which comprises three RNA molecules, particularly a crRNA molecule and a tracrRNA comprising at least two separate RNA molecules including a tracrRNA-A molecule and a tracrRNA-B molecule.
12) The gRNA of any one of items 11, wherein the locked hairpin forms a secondary structure comprising a contiguous stem having a length of at least 6 nt.
13) The gRNA of any one of items 1-12, wherein the locked hairpin has a secondary structure comprising a contiguous stem having a length of 6, 7, 8, 9 or 10 nt, particularly of 8 nt.
14) The gRNA of any one of items 1-13, wherein the locked hairpin has a secondary structure comprising a contiguous stem having at least 2 C-G base pairs.
15) The gRNA of any one of items 1-14, wherein the locked hairpin has a secondary structure comprising a contiguous stem having 2, 3, 4 or 5 C-G base pairs, particularly 4 C-G base pairs.
16) The gRNA of any one of items 1-15, wherein the locked hairpin has a secondary structure comprising a contiguous stem having a melting temperature of at least about 60°C, at least about 65°C or at least about 70°C.
17) The gRNA of any one of items 1-16, wherein the locked hairpin comprises a loop having a nucleotide sequence selected from:
   (i) 5'-GNRA-3',
   (ii) 5'-UNCG-3', and
   (iii) 5'- CUUG-3',
   wherein R is a nucleotide selected from A or G and N is any nucleotide e.g. selected from A, C, G, or U.
18) The gRNA of any one of items 1-17, wherein the locked hairpin comprises a loop having a nucleotide sequence selected from:
   (i) 5'-UUCG-3', and
   (ii) 5'-CUUG-3'.
19) The gRNA of any one of items 1-18, which comprises at least one modified nucleotide building block.
20) The gRNA of item 19, wherein the at least one modified nucleotide building block is selected from:
   (a) a nucleobase-modified building block,
   (b) a sugar-modified building block,
   (c) a backbone-modified building block,
   (d) a modified nucleotide building block having attached thereto a heterologous moiety, and
   (e) any combination thereof.
21) The gRNA of item 19 or 20, which comprises at least one nucleobase-modified building block comprising a non-RNA nucleobase, e.g. thymine.
22) The gRNA of any one of items 19-21, which comprises at least one sugar-modified building block comprising a non-ribose sugar moiety particularly selected from:
   (iv) a sugar-modified building block comprising a modified substituent at the 2'-position of the ribose moiety, particularly a substituent 2'-R wherein R is H, halo, e.g. F, Cl, Br, or I, C₁-C₅ alkyl, O-C₁-C₅ alkyl, S-C₁-C₅ alkyl, C₂-C₅ alkenyl, O-C₂-C₅ alkenyl, S-C₂-C₅ alkenyl, C₂-C₅ alkynyl, O-C₂-C₅ alkynyl, S-C₂-C₅ alkynyl or amino including mono- and disubstituted amino,
   (v) a sugar-modified building block, wherein two non-neighboring ring atoms of the ribose moiety are connected by a bridge, particularly a 2'-4'-bridged building block wherein the bridge typically has a length of 2-5 atoms, particularly 2-3 atoms including C-atoms and optionally heteroatoms such as O, N or S, or
   (vi) a sugar-modified building block wherein the ring of the ribose moiety is modified, e.g. a morpholino moiety, a thio-ribose moiety, or a 6-membered pyranose moiety.
23) The gRNA of any one of items 19-22, which comprises at least one sugar-modified nucleotide building block, wherein the substituent at the 2'-position of the ribose moiety is 2'-OCH₃.
24) The gRNA of any one of items 19-23, which comprises at least one sugar-modified nucleotide building block, which is a locked nucleic acid (LNA) building block.
25) The gRNA of any one of items 19-24, which comprises at least one backbone-modified building block comprising a modified internucleosidic linkage.
26) The gRNA of any one of items 19-25, which comprises at least one backbone-modified nucleotide building block comprising a phosphorothioate linkage.
27) The gRNA of any one of items 19-26, which comprises at least one modified nucleotide building block having attached thereto a lipid moiety or a polyethylene glycol moiety.
28) The gRNA of any one of items 19-27, wherein the at least one modified building block is present at a position, where it does not interact with a Cas enzyme.
29) The gRNA of any one of items 19-28, wherein the at least one modified building block is present in a portion of the gRNA selected from the target-specific spacer sequence, the repeat sequence, the anti-repeat sequence, and the hairpin sequence comprising a first hairpin and optionally a second hairpin.
30) The gRNA of any one of items 19-29, wherein at least one backbone-modified building block is present at a 5'-end and/or at a 3'-end of the gRNA, and particularly wherein at least two backbone-modified building blocks are present at a 5'-end and/or at a 3'-end of the gRNA.
31) The gRNA of any one of items 3-27, wherein the nexus does not comprise a modified nucleotide building block.
32) The gRNA of any one of items 1-18, which does not comprise any modified nucleotide building block.
33) A guide RNA (gRNA) suitable for CRISPR-mediated Oligonucleotide binding and/or editing, particularly according to any one of items 1-32, comprising a locked hairpin structure comprising the nucleotide sequence SEQ ID NO. 1:
   5'-X₁-XₙGGAC(N)ᵣGUCCXₙ₊₁-X₂ₙ-3'
   wherein X is any nucleotide e.g. selected from A, C, G, or U, with the proviso that X₁-Xₙ form a contiguous hairpin stem with Xₙ₊₁-X₂ₙ and n is 2, 3, 4, 5, or 6, particularly 3, 4 or 5, and more particularly 4;
   wherein Nᵣ is the loop of the locked hairpin; and N is any nucleotide e.g. selected from A, C, G, or U, and r is 3, 4, 5 or 6, particularly 4 or 5, and more particularly 4.
34) The gRNA of item 33 wherein the locked hairpin comprises the nucleotide sequence SEQ ID NO. 2:
   5'-ACUUGGAC(N)rGUCCAAGU-3'.
   wherein Nᵣ is the loop of the locked hairpin, N is any nucleotide particularly selected from A, C, G, or U, and r is 3, 4, 5 or 6, particularly 4 or 5, and more particularly 4.
35) The gRNA of item 33 or 34 having at least one feature as defined in any one of items 2-32.
36) The gRNA of any one of items 33-35, wherein the loop Nᵣ has a nucleotide sequence selected from:
   (i) 5'-GNRA-3',
   (ii) 5'-UNCG-3', and
   (iii) 5'- CUUG-3',
   wherein R is a nucleotide selected from A or G, and N is any nucleotide e.g. selected from A, C, G, or U.
37) The gRNA of any one of items 33-36, wherein the loop Nᵣ has a nucleotide sequence selected from:
   (i) 5'-UUCG-3', and
   (ii) 5'-CUUG-3'.
38) The gRNA of any one of items 33-37, wherein the locked hairpin comprises the nucleotide sequence SEQ ID NO. 3:
   5'-ACUUGGACUUCGGUCCAAGU-3'
39) The gRNA of any one of items 33-38, wherein the locked hairpin comprises at least one modified nucleotide building block, particularly at least one sugar-modified building block.
40) The gRNA of any one of items 33-39, wherein the locked hairpin comprises at least one modified nucleotide building block, particularly at least one sugar-modified building block in one or more or all of the nucleotides X₁-Xₙ and Xₙ₊₁-X₂ₙ according to SEQ ID NO. 1.
41) The gRNA of any one of items 33-40, wherein the locked hairpin does not comprise a modified nucleotide building block in one or more or all of the nucleotides GGAC(N)rGUCC according to SEQ ID NO. 1.
42) A guide RNA (gRNA) suitable for CRISPR-mediated Oligonucleotide binding and/or editing, particularly according to any one of items 1-41, which comprises the nucleotide sequence SEQ ID NO. 4: 
5'-
AGCAUAGCAAGUUAAAAUAAGGCUAGUCCGUUAUCAACUUGGACUUCGGUCC
AAGUGGCACCGAGUCGGUGCUUU-3'
 wherein A, C, G, and U are nucleotide building blocks including modified nucleotide building blocks.
43) A guide RNA (gRNA) suitable for CRISPR-mediated Oligonucleotide binding and/or editing, particularly according to any one of items 1-42, which comprises the nucleotide sequence SEQ ID NO. 5: 
5’-
mA*mG*mCmAmUmAmGmCmAmAGUUmAAmAAUAmAmGGCUAGUCmCGUUAm
UmCAAmCmUmUGGACUUCGGUCCmAmAmGmUmGGmCmAmCmCmGmAmGm
UmCmGmGmUmGmCmU*mU*mU-3’
 wherein A, C, G, and U are non-modified nucleotide building blocks, mA, mC, mG, and mU are sugar-modified nucleotide building blocks, particularly 2'-O-Methyl-modified building blocks and * denotes a modified internucleosidic linkage, particularly a phosphorothioate linkage.
44) A trans-activating CRISPR RNA (tracrRNA) suitable for CRISPR-mediated Oligonucleotide binding and/or editing comprising a constant gRNA sequence comprising at least one locked hairpin secondary structure at a position that does not interfere with Oligonucleotide binding and/or editing.
   A further aspect of the present invention relates to a
45) A trans-activating CRISPR RNA (tracrRNA) suitable for CRISPR-mediated Oligonucleotide binding and/or editing comprising:
   - a constant gRNA sequence comprising a CRISPR repeat/anti-repeat-structure or a portion thereof and at least one locked hairpin secondary structure at a position that does not interfere with Oligonucleotide binding and/or editing.
46) A trans-activating CRISPR RNA (tracrRNA) suitable for CRISPR-mediated Oligonucleotide binding and/or editing comprising:
   - a constant gRNA sequence comprising in 5' to 3' direction a (i) an CRISPR anti-repeat sequence, (ii) optionally a nexus sequence and (iii) at least one locked hairpin secondary structure.
47) The tracrRNA of item 42 comprising at least one of the features as defined in any one of items 2-3 and 6-46.
48) A nucleic acid molecule encoding a gRNA of any one of items 1-43 or a tracrRNA of any one of items 44-47 optionally in operative linkage with an expression control sequence.
49) A library comprising a plurality of gRNAs of any one of items 1-43 comprising different target-specific spacer sequences and a constant gRNA sequence, particularly a common constant gRNA sequence comprising at least one locked hairpin secondary structure at a position that does not interfere with Oligonucleotide binding and/or editing.
50) A complex comprising a Cas enzyme and a gRNA of any one of items 1-43 or a Cas enzyme and a tracrRNA of any one of items 44-47.
51) The complex of item 50, wherein the Cas enzyme is selected from Cas9, Cas12a, Cas12e, Cas12b, Cas12i, Cas12h, Cas12c, Cas12d, Cas12f, Cas12g, Cas12k, Cas12j, Cas13a, Cas13b, Cas13c, Cas13d, and Cas14 including recombinant variants thereof.
52) The complex of item 51, wherein the Cas enzyme is selected from Cas9 Cas14 including recombinant variants thereof.
53) *In vitro* or ex *vivo* use of a gRNA of any one of items 1-43, a tracrRNA of item 44 or 47, a gRNA- or tracrRNA-encoding nucleic acid molecule of item 48, a library of item 49, or a complex of any one of items 50-52 for CRISPR-mediated Oligonucleotide binding and/or editing, particularly for genome editing.
54) The use of item 49, for CRISPR-mediated Oligonucleotide binding and/or editing, particularly for genome editing.in a eukaryotic cell such as an animal cell, e.g. a mammalian cell including a human cell, or a plant cell.
55) A preparation comprising a gRNA of any one items 1-43, a tracrRNA of item 44 or 47, a gRNA- or tracrRNA-encoding nucleic acid molecule of item 48, a library of item 49, or a complex of any one of items 50-52 for use in therapy including human medicine and veterinary medicine.
56) Use of a gRNA of any one of items 1-43, a tracrRNA of item 44 or 47, a gRNA- or tracrRNA-encoding nucleic acid molecule of item 48, a library of item 49, or a complex of any one of items 50-52 for genome editing in a plant organism or a part thereof.

Further, the present invention shall be explained in more detail by the following Figures, Tables and Examples.

### Figure Legends

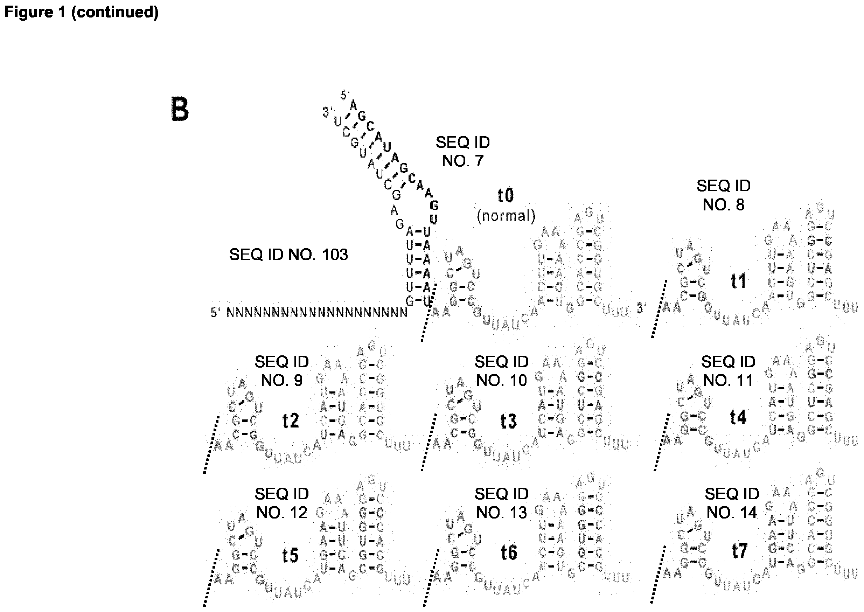

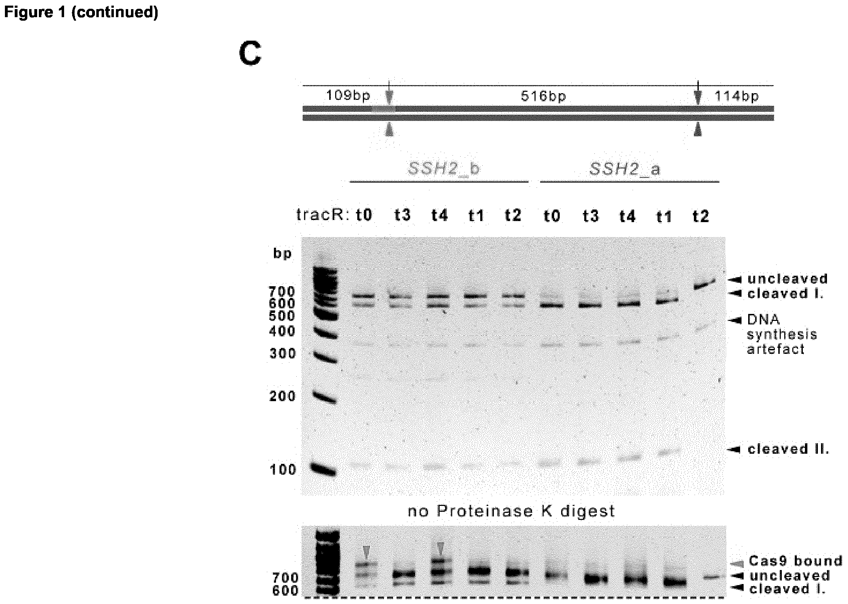

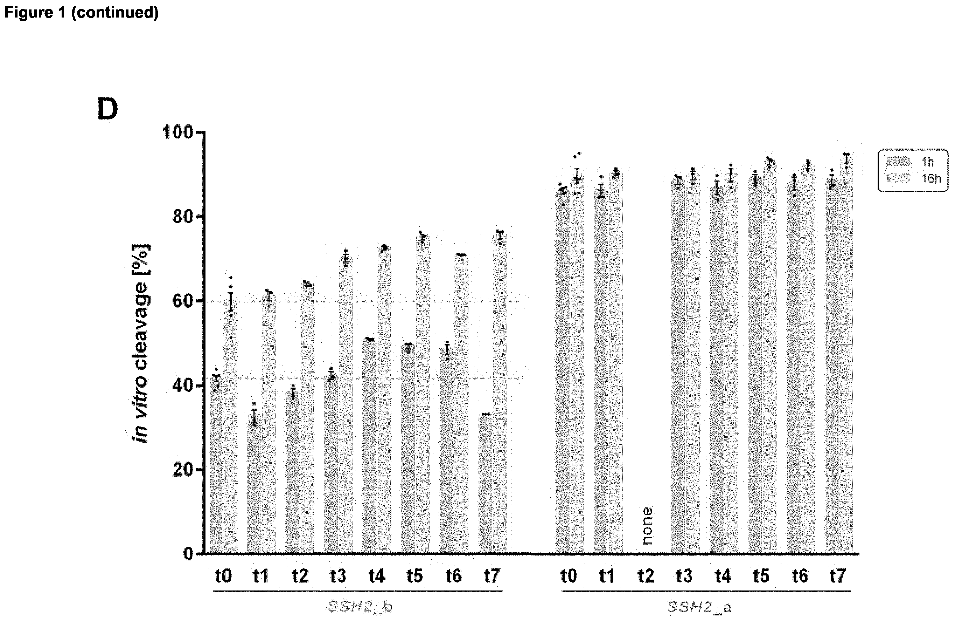

**Figure 1****: *In-vitro* cleavage efficiency of gRNAs with nucleotide swap modifications. (A)** gRNA (hybridized crRNA/tracR) from S. *pyogenes* for a canonically folded gRNA *(SSH2_a* gRNA) and a predicted non-canonical folded gRNA (SSH2_b gRNA) are shown. The target specific spacer sequence, as well as the structural motifs nexus, 1^{st} hairpin, and 2^{nd} hairpin are colored green/yellow, cyan, pink, and blue, respectively. **(B)** tracR sequences of the normal tracR (t0) and different nucleotide swap modifications (t1 to t7) are shown. The respective nucleotide swaps are colored red. **(C)** Exemplary gel picture after *in-vitro* cleavage of synthetic dsDNA with *SSH2_*a and *SSH2*_b gRNA target sites by gRNAs with different nucleotide swap designs. The bottom panel shows the related gel before proteinase K digest. Upwards shifted bands that are by presumably due to bound Cas9 are indicated with blue arrows. **(D)** Quantification of *in-vitro* cleavage efficiency after 1h (blue) and 16h (purple) for both *SSH2_a* and *SSH2*_b gRNA with different nucleotide swap modifications. Efficiencies of the normal tracR are shown with dotted lines for *SSH2*_b gRNA. Replicates are depicted by black dots and the error bars show the SEM. crRNAs, tracRs, and dsDNA target were chemically synthesized.

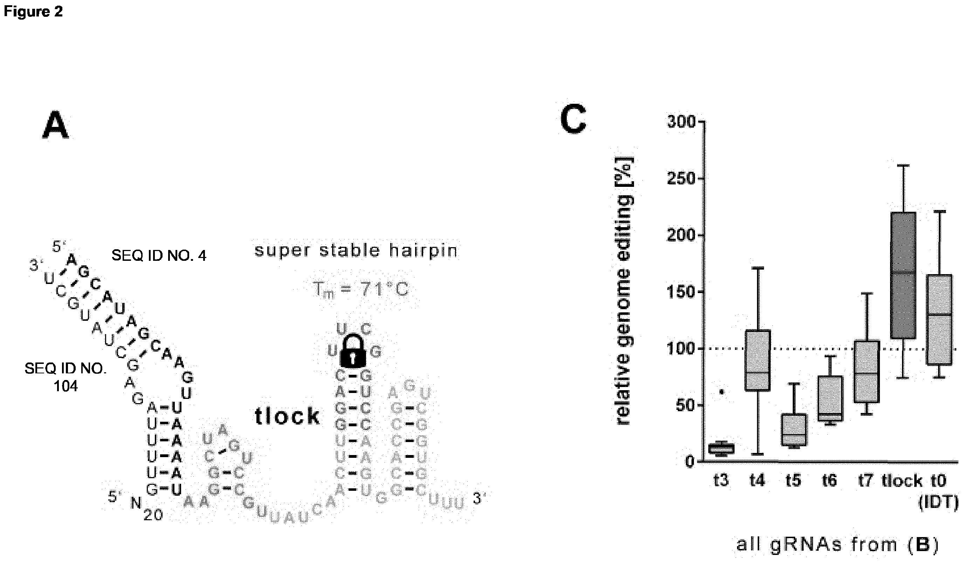

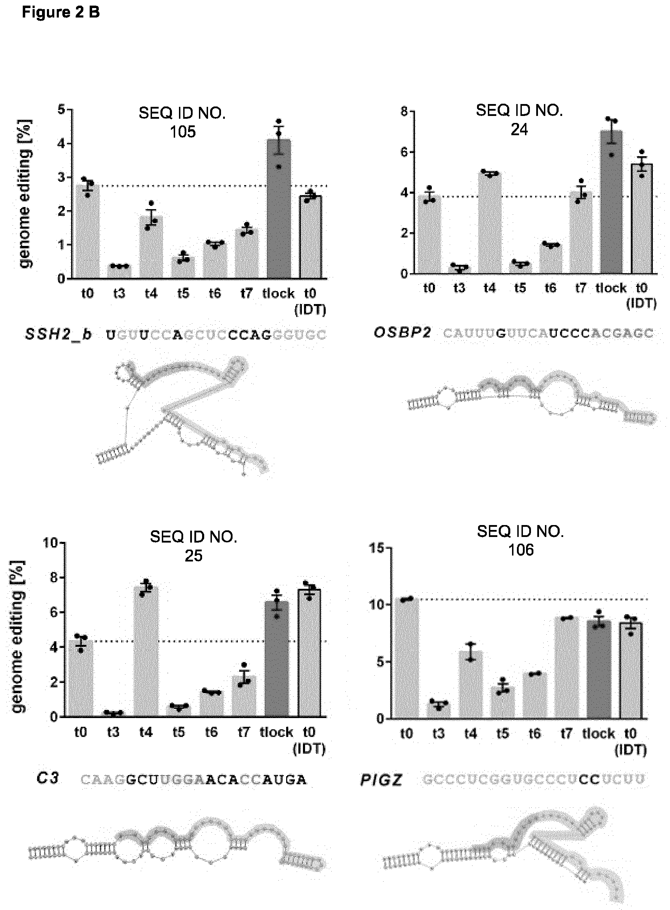

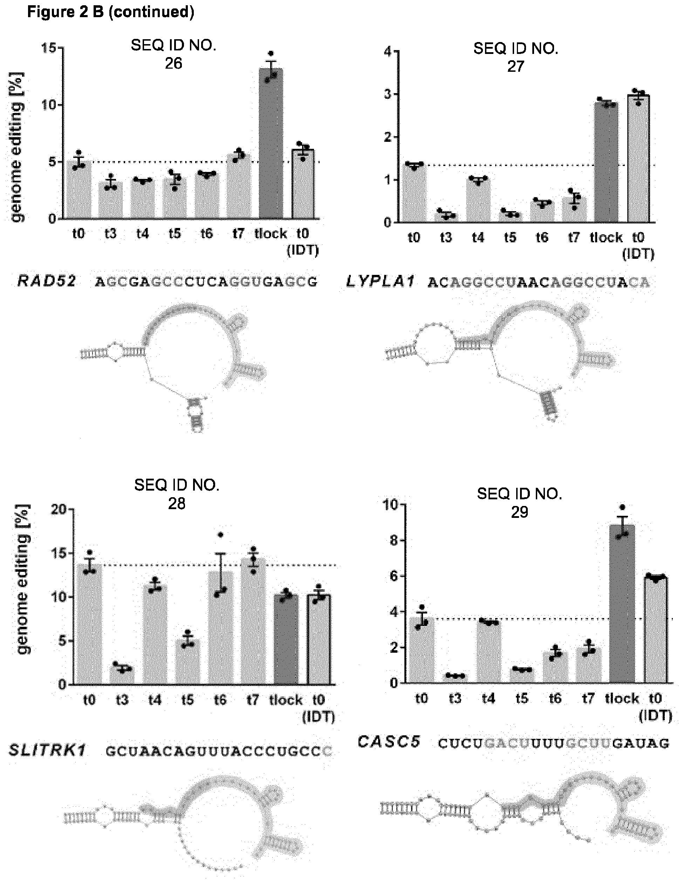

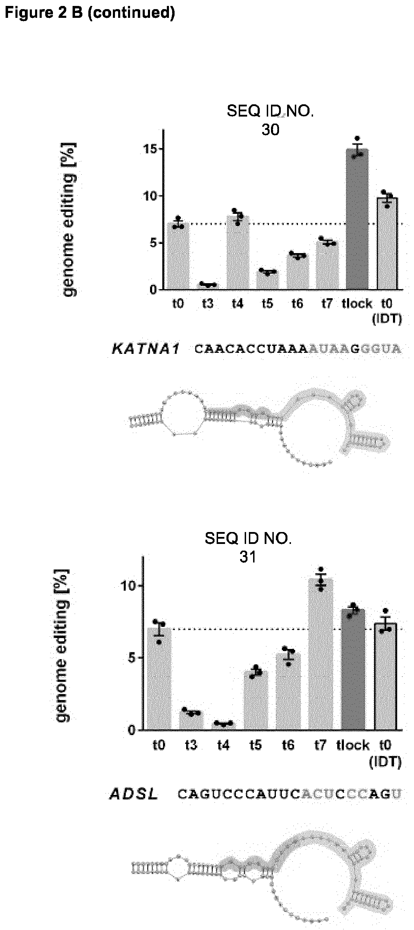

**Figure 2****: Genome editing efficiencies of gRNAs with different tracR backbone designs. (A)** 'Locked' tracR (tlock) gRNA design carrying an elongated 1^{st} hairpin with a superstable loop that should serve as a gRNA folding nucleation site and prevent misfolding. **(B)** Genome editing efficiencies for t0 (normal), t3, t4, t5, t6, t7, tlock (red), and commercial t0 (IDT) (black frame). t0 (IDT) that has the same nucleotide sequence as t0, but carries additional proprietary chemical modifications. Replicates are depicted by black dots and the error bars show the SEM. The panels show the efficiencies for 10 different gRNAs with different predicted non-canonical gRNA interactions to the nexus (cyan), 1^{st} hairpin (pink), 2^{nd} hairpin (blue), and/or the spacer sequence itself (brown). The predicted crRNA/tracR structure from the RNAstructure web server is shown below the spacer sequence. **(C)** Tukey box plots of relative genome editing efficiencies for different gRNA backbone designs for all gRNAs from B with respect the corresponding t0 tracR set to 100% are shown. Boxes extend from the 25th to 75th percentile and show the median as a line. Chemically synthesized gRNAs (hybridized crRNA/tracR) were lipofected into Cas9 expressing 409B2 iCRISPR human induced pluripotent stem cells (hiPSCs).

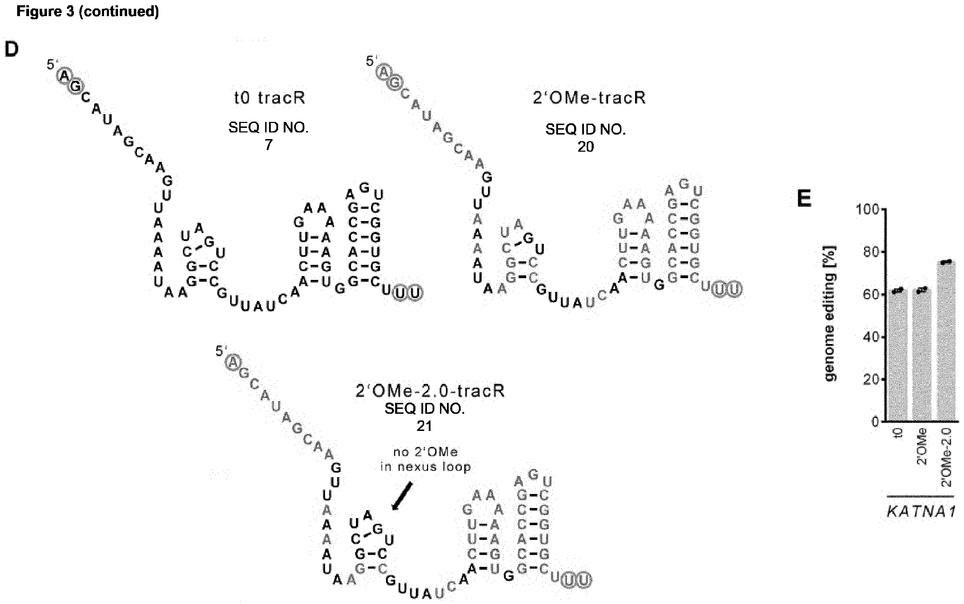

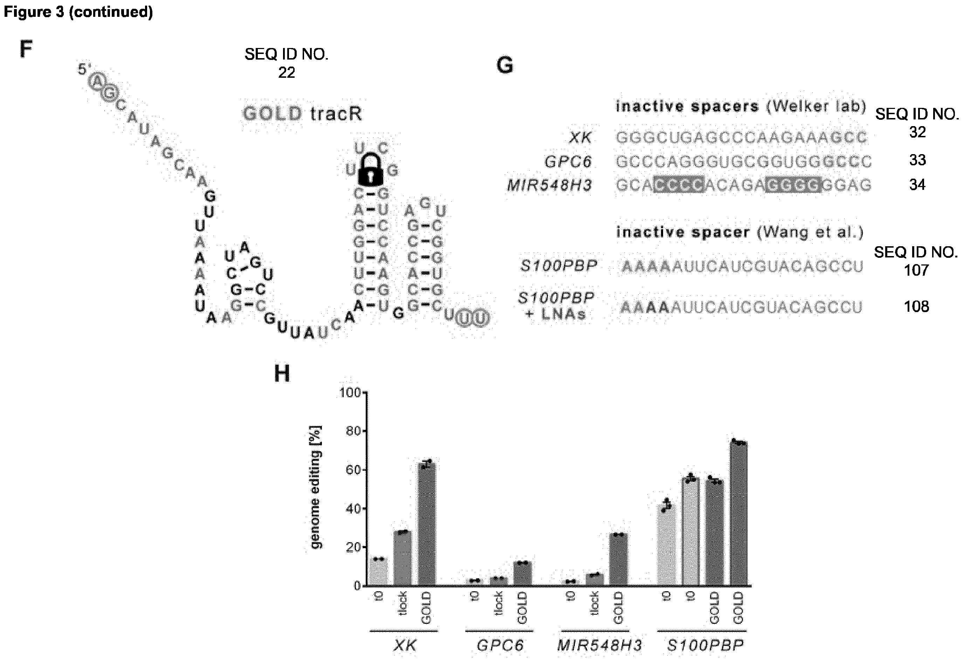

**Figure 3****: Optimization of chemical modifications of the gRNA to increase genome editing efficiency. (A)** Residues of the gRNA (hybridized crRNA/tracR) of which the 2'OH interacts with Cas9 or with residues of the nexus (cyan) are underlaid in red or circled in dark red, respectively. The residue underlaid in rose interacts with both Arg75 and Tyr52 of the bridge helix that modulates DNA cleavage. **(B)** Close-up of the crystal structure of the nexus and interacting residues adapted from NDB: 5F9R. Polar interactions are shown as dotted lines and those of 2'OH residues are shown as dotted red lines. **(C)** Single RNA residue structure and chemical modifications. **(D)** Chemical modifications of the normal tracR (t0), 2'OMe-tracR, and 2'OMe-2.0-tracR. **(E)** Genome editing efficiency of different chemically modified tracRs hybrized with a medium cleavage crRNA electroporated into Cas9 expressing 409B2 iCRISPR human induced pluripotent stem cells (hiPSCs). **(F)** Structure and chemical modifications of the genome editing optimized locked design (GOLD) tracR. **(G)** Spacer sequences of empirically determined inactive gRNAs. Putative cleavage inhibiting motifs are highlighted orange. LNA positions are colored brown. **(H)** Genome editing efficiency of normal tracR (blue), tlock tracR (red), and GOLD tracR (gold) hybridized with crRNAs from G after electroporation into Cas9 expressing 409B2 iCRISPR hiPSCs. Additional LNAs in the crRNA are indicated by a brown frame. Replicates are depicted by black dots and the error bars show the SEM.

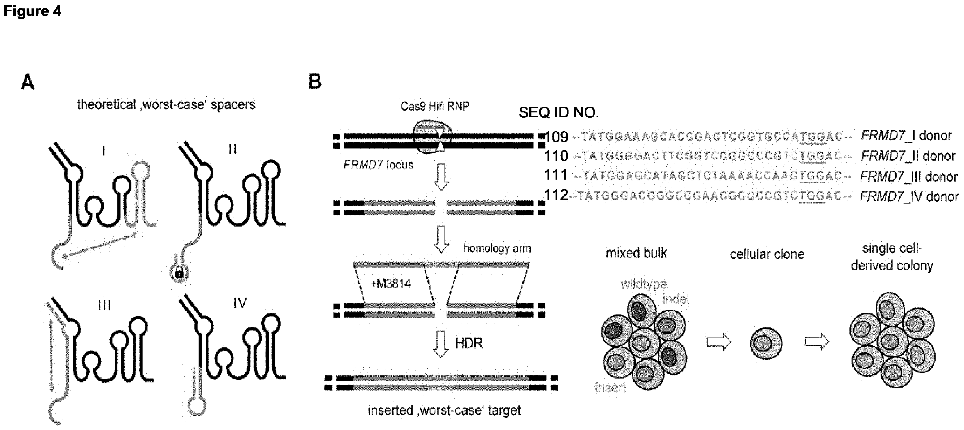

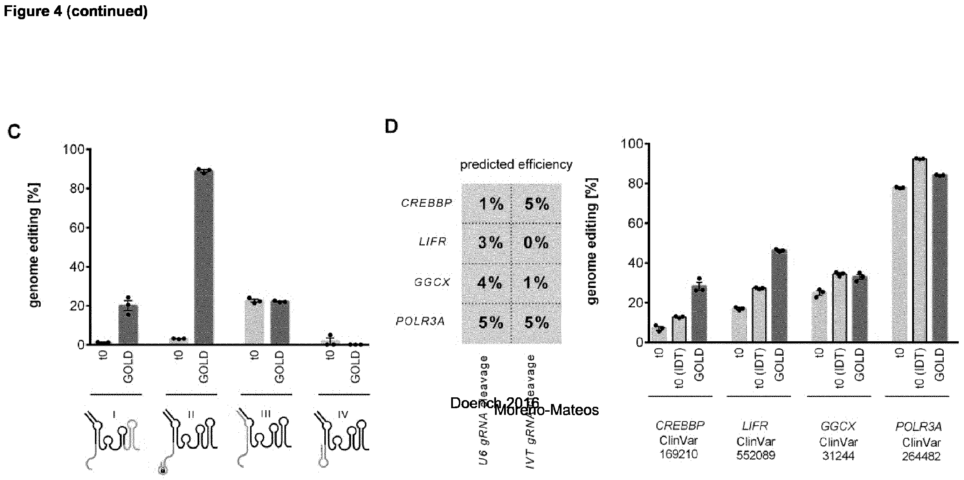

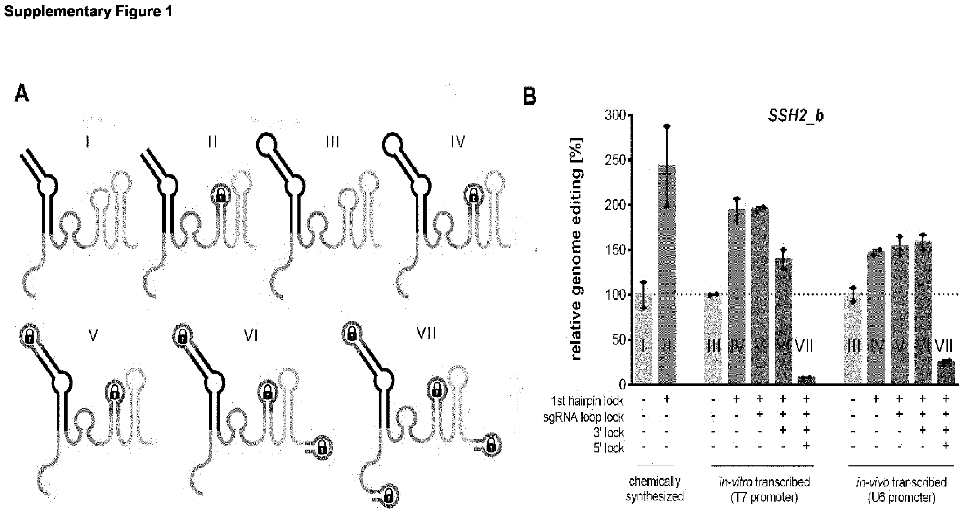

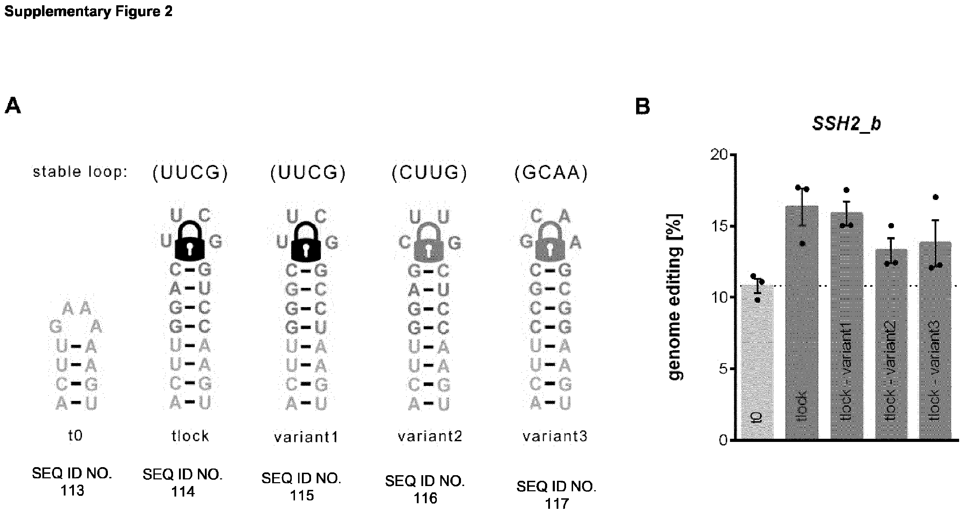

**Figure 4****: Genome editing efficiencies of predicted low performance gRNAs. (A)** Sketches of theoretical 'worst-case' spacers where the spacer would be perfectly complementary to the 3' end of the tracR (roman numeral I), form the unusually stable locked hairpin with itself (roman numeral II), be perfectly complementary to the 3' end of the crRNA (roman numeral III), or form a perfect hairpin along the whole spacer sequence (roman numeral IV). The complementary region is colored orange and expected interactions are presented as hairpins or indicated by red arrows. **(B)** Strategy to introduce the theoretical 'worst-case' spacer target sequences from C into 409B2 iCRISPR hiPSCs. Cleavage of the *FRMD7* locus by Cas9 Hifi RNP is followed by subsequent HDR using a ssODN donor sequence carrying the intended target site next to a TGG PAM. M3814 is added to block NHEJ and increase HDR efficiency. Four different edits are done for target insertions I-IV followed by generation of single cell derived cellular clones. **(C)** Genome editing efficiency of theoretical 'worst-case' gRNAs from C and t0 or GOLD tracR. gRNAs were electroporated into Cas9 expressing 409B2 iCRISPR hiPSCs that carry the corresponding inserted 'worst-case' target in the *FRMD7* locus. **(D)** Genome editing efficiency of gRNAs adjacent to a disease relevant ClinVar site and predicted to be self-complementary and to have very low cleavage efficiency (Doench 2016 and Moreno-Mateos 2015; percentile score both ≤ 5). Compared are t0 (normal), t0 (IDT) with proprietary chemical modifications, and GOLD tracR. Cas9 RNPs were electroporated into 409B2 hiPSCs. Replicates are depicted by black dots and the error bars show the SEM.

**Supplementary** **Figure 1****: Genome editing efficiencies of gRNAs carrying superstable hairpins at different positions. (A)** Design sketches of the gRNAs used in A labelled with roman numerals. Highlighted are the position of the 'locked' hairpin (dark red), spacer sequence (orange), nexus (cyan), 1^{st} hairpin (pink), and 2^{nd} hairpin (blue). **(B)** Relative genome editing efficiencies of chemically synthesized, *in-vitro* transcribed, and *in-vivo* transcribed gRNAs that carry 'locked' hairpins at different positions. Replicates are depicted by black dots and the error bars show the SEM. Oligonucleotides were electroporated into 409B2 iCRISPR human induced pluripotent stem cells (hiPSCs).
**Supplementary** **Figure 2****: Genome editing efficiencies of gRNAs with different locked hairpin variants. (A)** 1^{st} hairpin sequences of the normal tracR (t0), locked tracR (tlock), and three different locked tracR variants are shown. The elongated locked hairpin is colored red and nucleotide changes in the variants with superstable loops (UUCG, CUUG, GCAA) are orange. **(B)** Genome editing efficiencies of the crRNA/tracR hybrid gRNAs for the SSH2_b target. Replicates are depicted by black dots and the error bars show the SEM. The chemically synthesized tracRs additionally have two phosphorothioate bonds at both the 3' and 5' terminus to allow reasonable protection against degradation by nucleases and economic testing of different variants. Oligonucleotides were electroporated into 409B2 iCRISPR human induced pluripotent stem cells (hiPSCs).

**Supplementary Table 1: Oligonucleotides used in this study. Both RNA oligonucleotides (tracR and crRNA spacer sequence) as well as DNA oligonucleotides (primer, other ssDNA and dsDNA) are shown. Chemical modifications used are: *=phosphorothioate bond, mN=2'OMe, +=LNA.**

| | | |
|---|---|---|
| **tracR** | t0 (SEQ ID NO. 7) | AGCAUAGCAAGUUAAAAUAAGGCUAGUCCGU UAUCAACUUGAAAAAGUGGCACCGAGUCGGU GCUUU |
| | t1 (SEQ ID NO. 8) | AGCAUAGCAAGUUAAAAUAACGCUAGUCGGU UAUCAACUUGAAAAAGUGGCUCGGAGUCCGA GCUUU |
| | t2 (SEQ ID NO. 9) | AGCAUAGCAAGUUAAAAUAACGCUAGUCGGU UAUCAUCAUGAAAAUGAGGCACCGAGUCGGU GCUUU |
| | t3 (SEQ ID NO. 10) | AGCAUAGCAAGUUAAAAUAACGCUAGUCGGU UAUCAUCAUGAAAAUGAGGCUCGGAGUCCGA GCUUU |
| | t4 (SEQ ID NO. 11) | AGCAUAGCAAGUUAAAAUAAGGCUAGUCCGU UAUCAUCAUGAAAAUGAGGCUCGGAGUCCGA GCUUU |
| | t5 (SEQ ID NO. 12) | AGCAUAGCAAGUUAAAAUAAGGCUAGUCCGU UAUCAUGAAGAAAUUCAGCGUGGGAGUCCCA CGUUU |
| | t6 (SEQ ID NO. 13) | AGCAUAGCAAGUUAAAAUAAGGCUAGUCCGU UAUCAACUUGAAAAAGUGCGUGGGAGUCCCA CGUUU |
| | t7 (SEQ ID NO. 14) | AGCAUAGCAAGUUAAAAUAAGGCUAGUCCGU UAUCAUGAAGAAAUUCAGGCACCGAGUCGGU GCUUU |
| | tlock (SEQ ID NO. 4) | AGCAUAGCAAGUUAAAAUAAGGCUAGUCCGU UAUCAACUUGGACUUCGGUCCAAGUGGCACC GAGUCGGUGCUUU |
| | t0* (SEQ ID NO. 15) | A*G*CAUAGCAAGUUAAAAUAAGGCUAGUCCG UUAUCAACUUGAAAAAGUGGCACCGAGUCGG UGCU*U*U |
| | tlock* (SEQ ID NO. 16) | A*G*CAUAGCAAGUUAAAAUAAGGCUAGUCCG UUAUCAACUUGGACUUCGGUCCAAGUGGCAC CGAGUCGGUGCU*U*U |
| | tlock-variant1 * (SEQ ID NO. 17) | A*G*CAUAGCAAGUUAAAAUAAGGCUAGUCCG UUAUCAACUUGGGCUUCGGCCUAAGUGGCAC CGAGUCGGUGCU*U*U |
| | tlock-variant2* (SEQ ID NO. 18) | A*G*CAUAGCAAGUUAAAAUAAGGCUAGUCCG UUAUCAACUUGGAGCUUGCUCCAAGUGGCAC CGAGUCGGUGCU*U*U |
| | tlock-variant3* (SEQ ID NO. 19) | A*G*CAUAGCAAGUUAAAAUAAGGCUAGUCCG UUAUCAACUUCCGCGCAAGCGGAAGUGGCAC CGAGUCGGUGCU*U*U |
| | 2'OMe (SEQ ID NO. 20) | mA*mG*mCmAmUmAmGmCmAmAGUUmAAmAA UAmAmGmGmCmUmAGUmCmCGUUAmUmCAA |
| | | mCmUmUmGmAmAmAmAmAmAmGmUmGGmC mAmCmCmGmAmGmUmCmGmGmUmGmCmU* mU*mU |
| | 2'OMe-2.0 (SEQ ID NO. 21) | mA*mG*mCmAmUmAmGmCmAmAGUUmAAmAA UAmAmGGCUAGUCmCGUUAmUmCAAmCmUm UmGmAmAmAmAmAmAmGmUmGGmCmAmCm CmGmAmGmUmCmGmGmUmGmCmU*mU*mU |
| | GOLD-tracR (SEQ ID NO. 22) | mA*mG*mCmAmUmAmGmCmAmAGUUmAAmAA UAmAmGGCUAGUCmCGUUAmUmCAAmCmUm UGGACUUCGGUCCmAmAmGmUmGGmCmAmC mCmGmAmGmUmCmGmGmUmGmCmU*mU*m U |
| **crRNA spacer** | SSH2_ a _ alt-crRNA (SEQ ID NO. 23) | AGUGGCAUUCUGCUCAGAAU |
| | OSBP2_ alt-crRNA (SEQ ID NO. 24) | CAUUUGUUCAUCCCACGAGC |
| | C3_alt-crRNA (SEQ ID NO. 25) | CAAGGCUUGGAACACCAUGA |
| | RAD52_ alt-crRNA (SEQ ID NO. 26) | AGCGAGCCCUCAGGUGAGCG |
| | LYPLA1_alt-crRNA (SEQ ID NO. 27) | ACAGGCCUAACAGGCCUACA |
| | SLITRK1_alt-crRNA (SEQ ID NO. 28) | GCUAACAGUUUACCCUGCCC |
| | CASC5_alt-crRNA (SEQ ID NO. 29) | CUCUGACUUUUGCUUGAUAG |
| | KATNA1_alt-crRNA (SEQ ID NO. 30) | CAACACCUAAAAUAAGGGUA |
| | ADSL_alt-crRNA (SEQ ID NO. 31) | CAGUCCCAUUCACUCCCAGU |
| | XK _ alt-crRNA (SEQ ID NO. 32) | GGGCUGAGCCCAAGAAAGCC |
| | GPC6_alt-crRNA (SEQ ID NO. 33) | GCCCAGGGUGCGGUGGGCCC |
| | MIR548H3_alt-crRNA (SEQ ID NO. 34) | GCACCCCACAGAGGGGGGAG |
| | CREBBP_alt-crRNA (SEQ ID NO. 35) | AGCCUGGCAUGGGCUGCUGC |
| | LIFR_alt-crRNA (SEQ ID NO. 36) | CACACCGCUCAAAUGUUAUC |
| | GGCX alt-crRNA (SEQ ID NO. 37) | CUCCCCUUGCCCACCUCUGC |
| | POLR3A_alt-crRNA (SEQ ID NO. 38) | GUAAAUAAACUAACCCUUAU |
| | FRMD7_alt-crRNA (SEQ ID NO. 39) | GUGGGCUCUACAUAGCUAUG |
| | FRMD7_I_alt-crRNA (SEQ ID NO. 40) | AAAGCACCGACUCGGUGCCA |
| | FRMD7_II_alt-crRNA (SEQ ID NO. 41) | GGACUUCGGUCCGGCCCGUC |
| | FRMD7_III_alt-crRNA (SEQ ID NO. 42) | AGCAUAGCUCUAAAACCAAG |
| | FRMD7_IV_alt-crRNA (SEQ ID NO. 43) | GACGGGCCGAACGGCCCGUC |
| | S100PBP_full crRNA (SEQ ID NO. 44) | A*A*AAAUUCAUCGUACAGCCUGUUUUAGAGC UAUG*C*U |
| | S100PBP_full crRNA (+LNAs) (SEQ ID NO. 45) | A*A*+A+AAUUCAUCGUACAGCCUGUUUUAGAG CUAUG*C*U |
| **Primer** | In-vitro_target_F (SEQ ID NO. 46) | AAACGCCCTGTGTAGTGGTC |
| | In-vitro_target_R (SEQ ID NO. 47) | AGTACCCCAGGTTCTGCTCA |
| | U6_gBlock_F (SEQ ID NO. 48) | TGTACAAAAAAGCAGGCTTTAAA |
| | U6_gBlock_R (SEQ ID NO. 49) | TAATGCCAACTTTGTACAAGAAA |
| | SSH2_F (SEQ ID NO. 50) | ACACTCTTTCCCTACACGACGCTCTTCCGATCT AAGGGGAAGAGGGACTGCTT |
| | SSH2_R (SEQ ID NO. 51) | GTGACTGGAGTTCAGACGTGTGCTCTTCCGAT CTTGAGCAAAGGCAAAGGGAAA |
| | OSBP2_F (SEQ ID NO. 52) | ACACTCTTTCCCTACACGACGCTCTTCCGATCT AGTCTGCACCTACGCTGGTC |
| | OSBP2_R (SEQ ID NO. 53) | GTGACTGGAGTTCAGACGTGTGCTCTTCCGAT CTTCCTCCTTCCCTCTCCTTTG |
| | C3_F (SEQ ID NO. 54) | ACACTCTTTCCCTACACGACGCTCTTCCGATCT CAGATTCCCAGTGGATACGG |
| | C3_R (SEQ ID NO. 55) | GTGACTGGAGTTCAGACGTGTGCTCTTCCGAT CTTCCCAGCTCTGATTTGAACC |
| | RAD52_F (SEQ ID NO. 56) | ACACTCTTTCCCTACACGACGCTCTTCCGATCT GCTGCTTGGATTTTTGAACC |
| | RAD52_R (SEQ ID NO. 57) | GTGACTGGAGTTCAGACGTGTGCTCTTCCGAT CTCCGCTCTCGTGTTTGAGTTC |
| | LYPLA1_F (SEQ ID NO. 58) | ACACTCTTTCCCTACACGACGCTCTTCCGATCT AAAAACTGCTGTACACAAAAGCA |
| | LYPLA1_R (SEQ ID NO. 59) | GTGACTGGAGTTCAGACGTGTGCTCTTCCGAT CTTGTGTAGGTCTCAAGCAATTATCTG |
| | SLITRK1_F (SEQ ID NO. 60) | ACACTCTTTCCCTACACGACGCTCTTCCGATCT GGGCTTCAAATCAGCCAAG |
| | SLITRK1_R (SEQ ID NO. 61) | GTGACTGGAGTTCAGACGTGTGCTCTTCCGAT CTTTTCAAGACAAATGGGCAAG |
| | CASC5_F (SEQ ID NO. 62) | ACACTCTTTCCCTACACGACGCTCTTCCGATCT GGAAAAGCCTAGGAACACCA |
| | CASC5_R (SEQ ID NO. 63) | GTGACTGGAGTTCAGACGTGTGCTCTTCCGAT CTATGTTTTGCAAGTGGCTGA |
| | KATNA1_F (SEQ ID NO. 64) | ACACTCTTTCCCTACACGACGCTCTTCCGATCT CCTGACGGCAAAGGAATATAG |
| | KATNA1_R (SEQ ID NO. 65) | GTGACTGGAGTTCAGACGTGTGCTCTTCCGAT CTACTGTGCTTCCTTGTATTGTTGT |
| | ADSL_F (SEQ ID NO. 66) | ACACTCTTTCCCTACACGACGCTCTTCCGATCT AAAGTGTTCAAACGCCCTGT |
| | ADSL_R (SEQ ID NO. 67) | GTGACTGGAGTTCAGACGTGTGCTCTTCCGAT CTAGAGAGTACCCCAGGTTCTGC |
| | XK_F (SEQ ID NO. 68) | ACACTCTTTCCCTACACGACGCTCTTCCGATCT TCACCAAGAAGAGGCAAATG |
| | XK_R (SEQ ID NO. 69) | GTGACTGGAGTTCAGACGTGTGCTCTTCCGAT CTATTTTGATCCCCAAATGCAG |
| | GPC6_F (SEQ ID NO. 70) | ACACTCTTTCCCTACACGACGCTCTTCCGATCT GAAGTGCAGGAAAATGATGCT |
| | GPC6_R (SEQ ID NO. 71) | GTGACTGGAGTTCAGACGTGTGCTCTTCCGAT CTTGAGGGAGGAATTGGACATC |
| | MIR548H3_F (SEQ ID NO. 72) | ACACTCTTTCCCTACACGACGCTCTTCCGATCT GGCTTTTTATCCGCATTTGA |
| | MIR548H3_R (SEQ ID NO. 73) | GTGACTGGAGTTCAGACGTGTGCTCTTCCGAT CTGCAGACCAGGAACAGGAAAG |
| | S100PBP_F (SEQ ID NO. 74) | ACACTCTTTCCCTACACGACGCTCTTCCGATCT TCTTCTGGGGAAGATGATGG |
| | S100PBP_R (SEQ ID NO. 75) | GTGACTGGAGTTCAGACGTGTGCTCTTCCGAT CTCTGTTGGATTAAATGGTGCAA |
| | FRMD7_F (SEQ ID NO. 76) | ACACTCTTTCCCTACACGACGCTCTTCCGATCT TGCTCCTACCGCTAGTCCTG |
| | FRMD7_R (SEQ ID NO. 77) | GTGACTGGAGTTCAGACGTGTGCTCTTCCGAT CTGGTATTATGCCTCCCCAGGT |
| | CREBBP_F (SEQ ID NO. 78) | ACACTCTTTCCCTACACGACGCTCTTCCGATCT GAGCTGGGCGACTTCAGG |
| | CREBBP_R (SEQ ID NO. 79) | GTGACTGGAGTTCAGACGTGTGCTCTTCCGAT CTCCGACCCAACCAGGTGAG |
| | LIFR_F (SEQ ID NO. 80) | ACACTCTTTCCCTACACGACGCTCTTCCGATCT CCAAAAGTTCACTGATACCACCT |
| | LIFR_R (SEQ ID NO. 81) | GTGACTGGAGTTCAGACGTGTGCTCTTCCGAT CTTGTCTGCTGATTTCTCAACCTC |
| | GGCX_F (SEQ ID NO. 82) | ACACTCTTTCCCTACACGACGCTCTTCCGATCT GGGGACATGCACTAGAGGAA |
| | GGCX_R (SEQ ID NO. 83) | GTGACTGGAGTTCAGACGTGTGCTCTTCCGAT CTTTTACACAGAGTCGGCGATG |
| | POLR3A_F (SEQ ID NO. 84) | ACACTCTTTCCCTACACGACGCTCTTCCGATCT CAATGGGATCCACCACTTTT |
| | POLR3A_R (SEQ ID NO. 85) | GTGACTGGAGTTCAGACGTGTGCTCTTCCGAT CTGAGCCCAACAACTGATGTCC |
| **ssDNA** | FRMD7_I donor (SEQ ID NO. 86) | TATGCCTCCCCAGGTCTTTTTTTATGTGGACAA GCCACCCCCAGGTGCCCAGATGGTCCCCAATTA GAGCAGAGGAAAGGACAAGTCCATGGCACCG AGTCGGTGCTTTCATAGCTATGTAGAGCCCAC TGCAATGAAGCCAGCTGAA |
| | FRMD7_II donor (SEQ ID NO. 87) | TATGCCTCCCCAGGTCTTTTTTTATGTGGACAA GCCACCCCCAGGTGCCCAGATGGTCCCCAATTA GAGCAGAGGAAAGGACAAGTCCAGACGGGCC GGACCGAAGTCCCATAGCTATGTAGAGCCCAC TGCAATGAAGCCAGCTGAA |
| | FRMD7_III donor (SEQ ID NO. 88) | TATGCCTCCCCAGGTCTTTTTTTATGTGGACAA GCCACCCCAGGTGCCCAGATGGTCCCCAATTA GAGCAGAGGAAAGGACAAGTCCACTTGGTTTT AGAGCTATGCTCATAGCTATGTAGAGCCCACT GCAATGAAGCCAGCTGAA |
| | FRMD7_IV donor (SEQ ID NO. 89) | TATGCCTCCCCAGGTCTTTTTTTATGTGGACAA GCCACCCCAGGTGCCCAGATGGTCCCCAATTA GAGCAGAGGAAAGGACAAGTCCAGACGGGCC GTTCGGCCCGTCCATAGCTATGTAGAGCCCAC TGCAATGAAGCCAGCTGAA |
| | T7_promoter_IVT (SEQ ID NO. 90) | TAATACGACTCACTATAGG |
| | SSH2_b_IVT_III (SEQ ID NO. 91) | AAAGCACCGACTCGGTGCCACTTTTTCAAGTT GATAACGGACTAGCCTTAATTTAACTTGCTATG CTGTTTTCACAGCATAGCTCTAAATCGCACCCT GGGAGCTGGAACACCTATAGTGAGTCGTATTA |
| | SSH2_b_IVT_IV (SEQ ID NO. 92) | AAAGCACCGACTCGGTGCCACTTGGACCGAAG TCCAAGTTGATAACGGACTAGCCTTAATTTAAC TTGCTATGCTGTTTTCACAGCATAGCTCTAAAT CGCACCCTGGGAGCTGGAACACCTATAGTGAG TCGTATTA |
| | SSH2_b_IVT_V (SEQ ID NO. 93) | AAAGCACCGACTCGGTGCCACTTGGACCGAAG TCCAAGTTGATAACGGACTAGCCTTAATTTAAC TTGCTATGGGACCGAAGTCCCATAGCTCTAAAT CGCACCCTGGGAGCTGGAACACCTATAGTGAG TCGTATTA |
| | SSH2_b_IVT_VI (SEQ ID NO. 94) | GGACCGAAGTCCAAAGCACCGACTCGGTGCC ACTTGGACCGAAGTCCAAGTTGATAACGGACT AGCCTTAATTTAACTTGCTATGGGACCGAAGTC CCATAGCTCTAAATCGCACCCTGGGAGCTGGA ACACCTATAGTGAGTCGTATTA |
| | SSH2_b_IVT_VII (SEQ ID NO. 95) | GGACCGAAGTCCAAAGCACCGACTCGGTGCC ACTTGGACCGAAGTCCAAGTTGATAACGGACT AGCCTTAATTTAACTTGCTATGGGACCGAAGTC CCATAGCTCTAAATCGCACCCTGGGAGCTGGA |
| | | ACACCGGACCGAAGTCCTATAGTGAGTCGTAT TA |
| **dsDNA** | In-vitro target (SEQ ID NO. 96) | AAACGCCCTGTGTAGTGGTCTGCAAGGGCCAC GGGCGACTGCAGCTCCTCGGTGTCCCTGCTGT GGGTGAGCTGGTGACGTCTGTCTTCATGGAGT GGCATTCTGCTCAGAATGGGTGGAGACTATTG CTGCTTGATGGACAAAAGCAAACTTTTAAAACG GGAGAAGCCACAGGGTGGCTGAGGCTCTGAC TTTTGCTTGATAGAGGTTTCGCATGATTGAACA AGATGGGCTAACAGTTTACCCTGCCCTGGGCG GGATCTGTGCGCACGGATGGGAGGCTGGGCT GGCCGGGCGGGGACAAACAGCATATCTTTAAA TGGCTGCCAGTGCTTCTCCCTGCCTACATCCC GGGGCACAGAACATCCCAAGCCCTACTGGCCT GCCACGCTGTCGATCAGGGCCCAGGGTGCGG TGGGCCCGGGTCTCTGCCATGGTATCTAAACG GAGGCCGCTCGGGCCGCAGCGGGGAGAGAAT CATAAAAATGATATGGACCCGTCGCTATCGCT GCTGTGGTTATAGAACATACCCGTGAAGGGGA ACAAGATGGATTGCACGCAGGGCCACAACTGT GGTGGTTTTCGGGCAGTATACCAGTGCAATTG AGGTGTTCCAGCTCCCAGGGTGCTGGAAATTC TTTTAGGGCAGTCTGAATAGCTTCATCTTTTTC ACAATTAAGCTTCTGAATTAACTGTTCTCTGTCT TGCTCCTGGCTGCTGAGCAGAACCTGGGGTAC T |
| | SSH2_b_U6_III (SEQ ID NO. 97) | TGTACAAAAAAGCAGGCTTTAAAGGAACCAATT CAGTCGACTGGATCCGGTACCAAGGTCGGGC AGGAAGAGGGCCTATTTCCCATGATTCCTTCAT ATTTGCATATACGATACAAGGCTGTTAGAGAGA TAATTAGAATTAATTTGACTGTAAACACAAAGAT ATTAGTACAAAATACGTGACGTAGAAAGTAATA ATTTCTTGGGTAGTTTGCAGTTTTAAAATTATGT TTTAAAATGGACTATCATATGCTTACCGTAACTT GAAAGTATTTCGATTTCTTGGCTTTATATATCTT GTGGAAAGGACGAAACACCGGTGTTCCAGCTC CCAGGGTGCGATTTAGAGCTATGCTGTGAAAA CAGCATAGCAAGTTAAATTAAGGCTAGTCCGTT ATCAACTTGAAAAAGTGGCACCGAGTCGGTGC TTTTTTTCTAGACCCAGCTTTCTTGTACAAAGTT GGCATTA |
| | SSH2_b_U6_IV (SEQ ID NO. 98) | TGTACAAAAAAGCAGGCTTTAAAGGAACCAATT CAGTCGACTGGATCCGGTACCAAGGTCGGGC AGGAAGAGGGCCTATTTCCCATGATTCCTTCAT ATTTGCATATACGATACAAGGCTGTTAGAGAGA TAATTAGAATTAATTTGACTGTAAACACAAAGAT ATTAGTACAAAATACGTGACGTAGAAAGTAATA ATTTCTTGGGTAGTTTGCAGTTTTAAAATTATGT TTTAAAATGGACTATCATATGCTTACCGTAACTT GAAAGTATTTCGATTTCTTGGCTTTATATATCTT GTGGAAAGGACGAAACACCGGTGTTCCAGCTC |
| | | CCAGGGTGCGATTTAGAGCTATGCTGTGAAAA CAGCATAGCAAGTTAAATTAAGGCTAGTCCGTT ATCAACTTGGACTTCGGTCCAAGTGGCACCGA GTCGGTGCTTTTTTTCTAGACCCAGCTTTCTTG TACAAAGTTGGCATTA |
| | SSH2_b_U6_V (SEQ ID NO. 99) | TGTACAAAAAAGCAGGCTTTAAAGGAACCAATT CAGTCGACTGGATCCGGTACCAAGGTCGGGC AGGAAGAGGGCCTATTTCCCATGATTCCTTCAT ATTTGCATATACGATACAAGGCTGTTAGAGAGA TAATTAGAATTAATTTGACTGTAAACACAAAGAT ATTAGTACAAAATACGTGACGTAGAAAGTAATA ATTTCTTGGGTAGTTTGCAGTTTTAAAATTATGT TTTAAAATGGACTATCATATGCTTACCGTAACTT GAAAGTATTTCGATTTCTTGGCTTTATATATCTT GTGGAAAGGACGAAACACCGGTGTTCCAGCTC CCAGGGTGCGATTTAGAGCTATGGGACTTCGG TCCCATAGCAAGTTAAATTAAGGCTAGTCCGTT ATCAACTTGGACTTCGGTCCAAGTGGCACCGA GTCGGTGCTTTTTTTCTAGACCCAGCTTTCTTG TACAAAGTTGGCATTA |
| | SSH2_b_U6_VI (SEQ ID NO. 100) | TGTACAAAAAAGCAGGCTTTAAAGGAACCAATT CAGTCGACTGGATCCGGTACCAAGGTCGGGC AGGAAGAGGGCCTATTTCCCATGATTCCTTCAT ATTTGCATATACGATACAAGGCTGTTAGAGAGA TAATTAGAATTAATTTGACTGTAAACACAAAGAT ATTAGTACAAAATACGTGACGTAGAAAGTAATA ATTTCTTGGGTAGTTTGCAGTTTTAAAATTATGT TTTAAAATGGACTATCATATGCTTACCGTAACTT GAAAGTATTTCGATTTCTTGGCTTTATATATCTT GTGGAAAGGACGAAACACCGGTGTTCCAGCTC CCAGGGTGCGATTTAGAGCTATGGGACTTCGG TCCCATAGCAAGTTAAATTAAGGCTAGTCCGTT ATCAACTTGGACTTCGGTCCAAGTGGCACCGA GTCGGTGCTTTGGACTTCGGTCCTTTTTTTCTA GACCCAGCTTTCTTGTACAAAGTTGGCATTA |
| | SSH2_b_U6_VII (SEQ ID NO. 101) | TGTACAAAAAAGCAGGCTTTAAAGGAACCAATT CAGTCGACTGGATCCGGTACCAAGGTCGGGC AGGAAGAGGGCCTATTTCCCATGATTCCTTCAT ATTTGCATATACGATACAAGGCTGTTAGAGAGA TAATTAGAATTAATTTGACTGTAAACACAAAGAT ATTAGTACAAAATACGTGACGTAGAAAGTAATA ATTTCTTGGGTAGTTTGCAGTTTTAAAATTATGT TTTAAAATGGACTATCATATGCTTACCGTAACTT GAAAGTATTTCGATTTCTTGGCTTTATATATCTT GTGGAAAGGACGAAACACCGGACTTCGGTCC GGTGTTCCAGCTCCCAGGGTGCGATTTAGAGC TATGGGACTTCGGTCCCATAGCAAGTTAAATTA AGGCTAGTCCGTTATCAACTTGGACTTCGGTC CAAGTGGCACCGAGTCGGTGCTTTGGACTTCG GTCCTTTTTTTCTAGACCCAGCTTTCTTGTACA AAGTTGGCATTA |

### Examples

### 1. Methods

### 1.1 Cell culture

For this study we used the previously described iCRISPR-Cas9 line from 409-B2 human induced pluripotent stem cells (Riesenberg and Maricic, 2018) (GMO permit AZ 54-8452/26). Cells were grown on Matrigel Matrix (Corning, 35248) in mTeSR1 medium (StemCell Technologies, 05851) with supplement (StemCell Technologies, 05852) and MycoZap Plus-CL (Lonza, VZA-2011) that was replaced daily. For experiments in which Cas9 should be produced by the cell 2 µg/mL doxycycline (Clonetech, 631311) was added to the media three days in advance. At ∼ 80% confluency, adherent cells were dissociated using EDTA (VWR, 437012C) and split 1:6 to 1:10 in medium supplemented with 10µM Rho-associated protein kinase (ROCK) inhibitor Y-27632 (Calbiochem, 688000) for one day after replating. Cells were grown at 37°C in a humidified incubator with 5% CO₂. For generation of single cell derived cellular colonies (Fig. 1B), cells were dissociated and thoroughly separated using TrypLE Express (ThermoFisher, 12605036) and seeded in different dilutions.

### 1.2 Production of sgRNA

Secondary structure prediction of gRNAs was done using the RNAstructure web server (Bellaousov et al., 2013). Chemically synthesized crRNAs and tracRs as well as oligonucleotides for sgRNA production were ordered from IDT (Coralville, USA) (Supplementary Table 1). For production of sgRNAs by *in-vitro* transcription (IVT) ssDNA template were designed to contain the T7 promoter sequence in front of the sgRNA coding sequence. These ssDNA templates were hybridized with short complementary ssDNA for 2min at 95°C and let cooled down for 10min at 20°C to form a dsDNA T7 promoter. This template was used for IVT according to the manufacturer's protocol of the T7 High Yield RNA synthesis kit (NEB, E2040S). The IVT reaction mix was incubated overnight at 37°C before 2µL of TURBO DNase (Invitrogen, AM2238) was added and incubation continued for 30 additional minutes. IVT products were purified with the MEGAclear Transcription clean-up kit (Invitrogen, AM1908). For experiments in which sgRNAs are expressed in the cell, dsDNA fragments with a U6 promoter sequence in front of the sgRNA coding sequence were designed. Custom synthesized dsDNA fragments were amplified using Phusion HF MasterMix (Thermo Scientific, F-531L). The thermal cycling profile of the PCR was: 98 °C 30 s; 35 × (98° 10s, 61 °C 20s, 72 °C 25s); 72 °C 5 min. Successful amplifications was verified by size separating gel electrophoresis using EX agarose gels (Invitrogen, G4010-11) and PCR products were purified using Solid Phase Reversible Immobilization (SPRI) beads (Meyer and Kircher, 2010).

### 1.3 In-vitro cleavage of target DNA

The custom synthesized dsDNA target carrying cleavage sites for both gRNAs of interest was amplified using Phusion HF MasterMix (Thermo Scientific, F-531L) and purified using Solid Phase Reversible Immobilization (SPRI) beads (Meyer and Kircher, 2010). The thermal cycling profile of the PCR was: 98°C 30sec; 30x (98° 15sec, 60°C 30sec, 72°C 60sec); 72°C 5min. To form gRNAs, crRNAs were hybridized with tracR variants for 2 min at 95° and let cool down for 10 min at 20°C. For a 10 µl RNP complex solution 0.1 nmol gRNA was mixed with 63 pmol Cas9 (IDT) in 1x NEB 3.1 buffer (NEB, B7203S) and incubated for 20 min at 20°C. 5 µl of a 20 times diluted RNP complex solution was then added to 15 µl of a DNA target solution that contains 50 ng dsDNA target (Supplementary Table 1) in 1x NEB 3.1 buffer. After incubation at 37°C for 1 h or 1 6h the reaction was split in half, with one half being subjected to digestion with 0.3 µl of proteinase K (NEB, P8107S) for 30 min at 20°C. Subsequently, uncleaved target DNA and cleavage products were separated using size-separating gel electrophoresis in 4% EX agarose gels (ThermoFisher, G401004) and imaged with a Gel Jet Imager (Intas). Band intensities were quantified with ImageJ software.

### 1.4 Lipofection of oligonucleotides

Lipofection (reverse transfection) was done using the alt-CRISPR manufacturer's protocol (IDT) with a final concentration of 7.5 nM of each gRNA that had been previously formed by hybridization of crRNA and tracR. In brief, 0.75µl RNAiMAX (Invitrogen, 13778075) and the respective gRNAs were separately diluted in 25µl OPTI-MEM (Gibco, 1985-062) each and incubated at 20°C for 5 min. Both dilutions were mixed to yield 50 µl of OPTI-MEM including RNAiMAX and gRNAs. The lipofection mix was incubated for 20-30 min at 20°C. During incubation 409-B2 hiPS iCRISPR cells were dissociated using EDTA for 5 min and counted using the Countess Automated Cell Counter (Invitrogen). The lipofection mix, 100 µl containing 25,000 dissociated cells in mTeSR1 supplemented with Y-27632 and 2 µg/ml doxycycline were thoroughly mixed and put in 1 well of a 96-well plate covered with Matrigel Matrix (Corning, 35248). Media was exchanged to regular mTeSR1 media after 24 h.

### 1.5 Electroporation of oligonucleotides and RNPs

Electroporation of oligonucleotides was done using the B-16 program of the Nucleofector 2b Device (Lonza) in cuvettes for 100 µl Human Stem Cell nucleofection buffer (Lonza, VVPH-5022), containing 1 million cells, 78 pmol electroporation enhancer and 320 pmol of gRNA. For generation of 'worst-target' inserted cells (Fig. 4B) or RNP based editing of ClinVar positions (Fig. 4D) 200 pmol of single stranded DNA donor (Supplementary Table 1) or 252 pmol Cas9 (IDT) were additionally added to the nucleofection buffer, respectively. No doxycycline was added to the media of cells that were used for RNP based editing. For generation of HDR-edited cells the small molecule M3814 (MedChemExpress, HY-101570 ) was added to the media for two days post-electroporation to increase HDR efficiency (Riesenberg et al., 2019).

### 1.6 Illumina library preparation and sequencing

At least five days after transfection cells were detached using TrypLE (ThermoFisher, 12605036), pelleted, and resuspended in 15 µl QuickExtract (Lucigen, QE0905T). Incubation at 65°C for 10 min, 68°C for 5min and 98°C for 5 min was performed to yield single stranded DNA as a PCR template. Primers for each targeted loci were designed to contain adapter overhangs for Illumina sequencing (Supplementary Table 1). PCR was done in a T100 Thermal Cycler (Bio-Rad) using the KAPA2G Robust PCR Kit (SIGMA, KK5024) with buffer B and 3 µl of cell extract in a total volume of 25 µl. The thermal cycling profile of the PCR was: 95°C 3 min; 34x (95° 15 sec, 65°C 15 sec, 72°C 15 sec); 72°C 60 sec. P5 and P7 Illumina adapters with sample specific indices were added in a subsequent PCR reaction (Kircher et al., 2012) using Phusion HF MasterMix (Thermo Scientific, F-531L) and 0.3 µl of the first PCR product. The thermal cycling profile of the second PCR was: 98°C 30 sec; 25x (98° 10 sec, 58°C 10 sec, 72°C 20 sec); 72°C 5 min. Amplifications were verified by size separating agarose gel electrophoresis using 2% EX gels (Invitrogen, G4010-11). The indexed amplicons were purified using Solid Phase Reversible Immobilization (SPRI) beads (Meyer and Kircher, 2010). Double-indexed libraries were sequenced on a MiSeq (Illumina) giving paired-end sequences of 2 x 150 bp (+7 bp index). After base calling using Bustard (Illumina) adapters were trimmed using leeHom (Renaud et al., 2014).

### 1.7 Amplicon sequence analysis

Bam-files were demultiplexed and converted into fastq files using SAMtools (Li et al., 2009). CRISPResso (Pinello et al., 2016) was used to analyse fastq files for percentage of wildtype and indel sequences. For analysis of precise insertion frequency of 'worst-case' targets in single cell derived cellular clones, the expected amplicon was provided to CRISPResso to call the HDR event. Analysis was restricted to amplicons with a minimum of 70% similarity to the wild-type sequence and to a window of 20 bp from each gRNA. Unexpected substitutions were ignored as putative sequencing errors.

### 2. Results

### 2.1 In-vitro nucleotide-swap tracR

First, we aimed to solve the issue of internal misfolding which can occur if the spacer sequence has partial complementarity to the sgRNA backbone or tracR and thus results in non-canonical gRNA secondary structures. Thyme et al. could activate an inactive sgRNA *in-vitro* by breaking the predicted unwanted non-canonical interaction with a base substitution in the sgRNA backbone (Thyme et al., 2016). However, generalization of this approach is hampered by inaccuracies of RNA-folding prediction (Wang et al., 2019b) and the need for spacer specific custom modification of the normally constant part of the sgRNA backbone or tracR, when sgRNA or crRNA/tracR duplex are used to provide gRNA, respectively. We hypothesized that engineering maximum sequence distance of the tracR from its original would generate a universal backup tracR for inactive spacers, since misfolding of certain gRNAs is due to partial complementary of the spacer to the tracR. We searched for gRNA positions that do not interact with the Cas9 protein (Nishimasu et al., 2014) and are thus amenable for substitutions and designed four different tracRs with swaps of opposing nucleotides in the nexus, 1^{st} hairpin, and 2^{nd} hairpin and tested the *in-vitro* cleavage efficiency of corresponding crRNA/tracR gRNAs for a high cleavage crRNA and low cleavage crRNA predicted to have non-canonical hybridization between the spacer and the 2^{nd} hairpin (Fig. 1A and B). Modification of the nexus reduces Cas9 binding to the target DNA (Fig. 1C), can reduce cleavage, and totally abrogated cleavage for one experimental condition, while a tracR with original nexus and two nucleotide swaps in each the 1^{st} and 2^{nd} hairpin increased cleavage of the low cleavage crRNA and had no effect on cleavage of the high cleavage gRNA (Fig. 1D). A complete nucleotide swap of the 1^{st} hairpin, and both the 1^{st} and 2^{nd} hairpins similarly increase cleavage of the low cleavage crRNA and are not detrimental to cleavage of the high cleavage crRNA (Fig. 1A and D).

### 2.2 In-vivo nucleotide-swap and locked tracR

Next, we wanted to confirm the *in-vitro* effect of nucleotide swap modifications of the tracR in genome editing of cells, because of the frequent discrepancy of *in-vitro* cleavage and *in-vivo* cleavage efficiency (Briner et al., 2014). In addition to tested nucleotide swap tracRs we also tested a completely different approach in which the 1^{st} hairpin is elongated with an unusually stable RNA hairpin with a melting temperature of 71°C (Varani et al., 1991), which we name 'locked hairpin' tracR (Fig. 2A). We hypothesized that the unusually stable hairpin would serve a nucleation site for RNA folding and thus acts as molecular spring that can disentangle misfolded gRNA regardless of the spacers sequence composition. We used the previously described 409B2 human induced pluripotent stem cell (hiPSC) line carrying doxycycline inducible Cas9 (iCRISPR-Cas9) (Riesenberg and Maricic, 2018) to test combinations of the different tracR designs and ten different crRNAs with most of them having predicted strong non-canonical interactions to different regions in the gRNA (Fig. 2). Cas9 expressing cells were lipofected with chemically synthesized gRNAs for 24 h, DNA was extracted after two additional days, followed by target amplification PCR, and subsequent Illumina sequencing to determine genome editing efficiencies. In line with *in-vitro* data nexus modification reduces genome editing efficiency *in-vivo,* but *in-vivo* data lacks a clear causal effect of nucleotide swaps that would break *in-silico* predicted (Bellaousov et al., 2013) non-canonical interactions, and nucleotide swap modifications could only increase genome editing efficiencies in few cases. The locked tracR increased genome editing efficiency for eight of ten targets irrespective of the position of the predicted non-canonical interaction (Fig. 2B). The mean relative cleavage efficiency across targets increased to 169% (ranging from 75% to 262%) with respect to use of the normal tracR (Fig. 2C).

To confirm the efficacy of locked hairpins to increase genome editing efficiency independent of gRNA delivery and format we designed double-stranded DNA carrying a T7 or U6 promoter followed by sequences coding for sgRNAs with HEAT modifications that carry locked hairpins at different positions (Supplementary Fig. 1A). DNA templates with a T7 promoter were used for *in-vitro* transcription (IVT) of sgRNAs. Chemically synthesized crRNA/tracR duplex gRNAs, IVT sgRNAs or DNA templates with a U6 promoter that allows *in-vivo* transcription of sgRNAs were electroporated into Cas9 expressing cells. Elongation of the 1^{st} hairpin with the hairpin lock increased relative cleavage efficiency for chemically synthesized crRNA/tracR duplex gRNA, IVT sgRNA, and *in-vivo* transcribed sgRNA (Supplementary Fig. 1B). Addition of a hairpin lock in the artificial sgRNA fusion loop did not change cleavage efficiency. Even further addition of a locked hairpin at both the 3' and 5' almost abrogated cleavage, while addition at the 3' end only decreased efficiency of IVT sgRNA. We also tested other locked designs that carry hairpin loop sequences (UUCG), (GCAA), or (CUUG), described to form hyperstable noncanonical stabilizing interactions (Chen and Garcia, 2013) (Supplementary Fig. 2A). All tested locked designs increased genome editing efficiency (Supplementary Fig. 2B).

### 2.3 Improved chemical modifications and LNAs

After having established the efficacious locked hairpin tracR design we sought to maximize its efficiency by further chemical modification (Hendel et al., 2015; Yin et al., 2017). Yin et al. described an enhanced e-sgRNA with phosphorothioate bonds and ample internal 2'OMe modification (76% of tracR residues carry 2'OMe). These include modified residues in the nexus in which an opposing nucleotide swap decreased cleavage efficiency in our study, which hints to a potential detrimental effect of nexus modifications. When evaluating the crystal structure of Cas9 primed for cleavage (Jiang et al., 2016) we found that 2'OH groups of residues in the nexus exhibit direct polar contacts in the nexus itself and could thus possibly stabilize the productive state of the Cas9 ribonucleoprotein (Fig. 1A and B). To test different chemical modifications (Fig. 1C) of gRNA, we electroporated medium efficiency crRNA duplexed with a tracR carrying sole phosphorothioate end protection, phosphorothioate end protection and additional 2'OMe adapted from *Yin et al.,* and the latter but without 2'OMe modifications in the nexus loop (Fig. 3D) into Cas9 expressing cells. We also did not modify a nexus-proximal uracil that interacts with Arg75 and Tyr72 of the Cas9 bridge helix, which has been described to modulate DNA cleavage (Babu et al., 2019).

Indeed, refraining from 2'OMe modifications of the nexus increased absolute genome editing efficiency from 62 to 75% for the tested medium efficiency gRNA, while no increase was achieved when the nexus carried 2'OMe modifications (Fig. 3E). Both further optimized chemical modification of a gRNA by phosphorothioate end protection and internal 2'OMe modifications that do not include the nexus loop, as well as an engineered gRNA backbone with a locked 1^{st} hairpin can increase genome editing efficiency. We thus combined both improvements into a genome editing optimized locked design (GOLD) - tracR (Fig. 3F), and tested the GOLD-tracR with three crRNAs containing spacers that were unable to cleave in both human and *E.coli* cells in a plasmid based screen (Talas et al., 2021). Two of these inactive spacers contain a terminal PAM-proximal GCC motif, which has been previously described to abrogate cleavage (Graf et al., 2019) (Fig. 3G). The GOLD-tracR could increase genome editing efficiency for all these spacers more than the locked tracR and increased the relative genome editing efficiency to 676% (from 434% to 1146%), with respect to the normal tracR (Fig. 3H). Mean absolute genome editing efficiency across spacers could be increases from 6% to 34%. We further used the GOLD-tracR together with a crRNA containing the spacer that had been described to have the lowest genome editing efficiency in a ∼50.000 U6 promoter expressed gRNA screen (Wang et al., 2019a). We also tested the crRNA with terminal locked nucleic acids (LNAs), as its seven terminal spacer residues are normally only able to form two hydrogen bonds each and low terminal melting temperature in the spacer has been associated with reduced cleavage efficiency (Wang et al., 2019a). LNAs are commonly used oligonucleotide modifications that increase the melting temperature. Interestingly, the spacers target was not intractable with standard CRISPR-Cas9 editing using chemically synthesized gRNA in our hands, and terminal LNAs moderately increased absolute genome editing efficiency from 42% to 55% when using a normal tracR and the GOLD-tracR further increased efficiency to 73% (Fig. 3H).

### 2.4 Predicted bad loci and worst-case targets

To further evaluate robustness of the GOLD-tracR to increase genome editing, we aimed to test it on theoretical 'worst-case' DNA targets for which the corresponding gRNA spacer I) is perfectly complementary to the 3' end of the tracR gRNA, II) in which the spacer forms the unusually stable locked hairpin (Varani et al., 1991) with itself, III) in which the spacer is perfectly complementary to the 3' end of the crRNA, or IV) forms a perfect hairpin along the whole spacer sequence (Fig. 4A). The target II also carries an inhibitory PAM-proximal GCC motif (Graf et al., 2019). Since those four 'worst-case' targets do not exist in the human genome we introduced each of them in the genome of Cas9 expressing cells by supplying a DNA donor for HDR and clonal isolation of cells carrying a 'worst-case' target (Fig. 4B). We then electroporated corresponding gRNAs (hybridized crRNA/tracR) into the respective generated cell lines. Theoretical 'worst-case' target/gRNA pairs I, II, and IV are refractory to editing with the normal tracR (t0), and III results in moderate efficiency (23%) (Fig. 4C). The GOLD-tracR is able to increase editing efficiency from 1.2% to 20% for I and from 3.2% to 89% for II, but does not increase efficiency for III and IV.

Finally, we benchmark the efficacy of the GOLD-tracR to a commercial chemically modified tracR (IDT) on editing efficiency of predicted hard-to-target disease relevant ClinVar loci (Landrum et al., 2018) related to Rubinstein-Taybi syndrome *(CREBBP),* Stueve-Wiedemann syndrome *(LIFR),* pseudoxanthoma elasticum-like disorder (GGCX), and Wiedemann-Rautenstrauch syndrome (POLR3A) with Cas9 ribonucleoprotein (RNP) electroporation. The gRNAs are predicted to have very low cleavage efficiency (percentile of ≤ 5% for both the Doench et al. 2016 (Doench et al., 2016) and the Moreno-Mateos et al. prediction score (Moreno-Mateos et al., 2015)) and to be self-complementary as judged by RNA-folding prediction (Bellaousov et al., 2013). The genome editing efficiency with the normal tracR is below 25% for three gRNAs and can thus be considered inefficient, but is 78% for the fourth target, underlining the inaccuracy of prediction algorithms even when different ones are combined (Fig. 4D). Both IDT and GOLD-tracR increase the relative genome editing efficiency for all targets with respect to the normal tracR (150% and 227%). The GOLD-tracR outperforms the commercial tracR with proprietary chemical modification almost 2-fold for the two least efficient gRNAs and has a comparable or only slightly lower efficiency for the other two gRNAs.

### 3. Discussion

It has been described that inactive gRNAs can contain hairpins in the spacer portion or interactions between the spacer portion and the backbone of the gRNA (Thyme et al., 2016). The same study showed that the predicted unwanted interaction can be broken by a base substitution in the interacting backbone sequence and allow *in-vitro* cleavage, and that heating and refolding can increase efficiency of misfolded gRNAs. However, sequence context dependent base substitutions require a different custom gRNA backbone change for every spacer sequence of interest, and refolding by heating is not applicable for CRISPR screens where gRNAs are expressed in cells. To find a related generalizable solution, we aimed to generate a universal backup tracR with maximum sequence distance to its original by nucleotide-swaps in gRNA regions that do not interact with the Cas9 protein. While a completely swapped first and second hairpin can increase cleavage efficiency of a gRNA *in-vitro,* this is not the case *in-vivo* (Fig. 1 and 2). Some nucleotide-swaps increase *in-vivo* editing efficiency for certain gRNAs. Unfortunately, the occasional beneficial effect of nucleotide-swaps cannot be inferred from the respective *in-silico* predicted interactions, which could be due to inaccuracy of RNA-folding prediction (Wang et al., 2019b).

Another approach to provide a generalizable solution to unintended RNA interactions was the introduction of an unusually stable hairpin (Varani et al., 1991) that should provide a RNA folding nucleation site and thus prevent misfolding. Hairpins with the C(UUCG)G loop motif are considered the most stable RNA hairpins and reverse transcriptase cannot read through this loop because of this (Tuerk et al., 1988). We elongated the 1^{st} hairpin with a hairpin containing the C(UUCG)G loop and dubbed the newly formed structure 'locked hairpin'. We chose to modify the 1^{st} hairpin, because it can be completely removed with only little loss in activity (Briner et al., 2014). Recently, it was further shown that Cas9 can tolerate elongation of the 1^{st} hairpin and also the sgRNA fusion loop, while modification of the 2^{nd} hairpin is detrimental for cleavage (Mullally et al., 2020).

Strikingly, the 'locked hairpin' tracR increases *in-vivo* genome editing efficiencies for eight of ten tested targets irrespective of the position of the predicted non-canonical interaction (Fig. 2). It increases efficiency irrespective of gRNA origin (chemically synthesized, *in-vitro* transcribed, *in-vivo* transcribed) and delivery method of oligonucleotides (lipofection, electroporation) (Fig. 2, Supplementary Fig. 1). Different superstable RNA tetraloops can be used to increase genome editing efficiency (Supplementary Fig. 2). The 'locked hairpin' is thus a gRNA modification that robustly increases genome editing efficiency irrespective of spacer sequence composition.

The commercially available tracR (IDT) also increased *in-vivo* genome editing efficiency for the majority of targets, albeit not as strong as the 'locked hairpin' tracR (Fig. 2). The nucleotide sequence of the commercial tracR is identical to the normal tracR, but it also carries proprietary chemical modifications. We thus sought to combine the 'locked hairpin' design with terminal phosphorothioate and internal 2'OMe modifications to further increase genome editing efficiency. The nexus has the highest level of conservation in the gRNAs of 41 *Streptococcus* and *Lactobacillus* genomes (Briner et al., 2014). In line with this observation, a nucleotide-swap in the nexus reduced Cas9 binding to the gRNA and cleavage efficiency in our experiments (Fig. 1 and 2), suggesting that nexus modifications are not well tolerated. When examining the crystal structure of CRISPR-Cas9 in complex with sgRNA and double-stranded DNA primed for target DNA cleavage (NDB: 5F9R) (Jiang et al., 2016) we find that 2'OH groups of residues in the nexus exhibit direct polar contacts in the nexus itself, which might be beneficial for cleavage (Fig. 3B). Consequently, 2'OH modification of these residues might be detrimental for cleavage. For one exemplary target we tested, the genome editing efficiency with a tracR that carries terminal phosphorothioates and internal 2'OMe modifications is indeed higher when the nexus does not carry 2'OMe modifications (Fig. 3E).

The overall absolute editing efficiency of the first *in-vivo* tracR screen in which gRNAs were transfected with lipofection (Fig. 2) was relatively low compared to subsequent experiments done using transfection by electroporation (Fig. 3 and 4). For example, the *KATNA1* gRNA with the normal tracR achieved 7% and 62% genome editing for lipofection and electroporation, respectively. On top of differences in transfection efficiency we attribute this difference to NOE by use of electroporation enhancer DNA (external non-homologous DNA NOE) in the latter, which is thought to divert cells towards error-prone instead of error-free repair pathways thus resulting in higher measured genome editing efficiencies (Richardson et al., 2016). However, even when electroporation with NOE is employed, gRNAs with spacers previously tested to be ineffective in human and *E. coli* (Talas et al., 2021) are ineffective in our hands as well (Fig. 3H). Strikingly, combining the locked design with chemical modifications into the genome optimized locked design (GOLD)-tracR enables reasonable editing efficiencies of these complicated refractory targets.

To test the universal applicability of the GOLD-tracR we generated cell lines with theoretical worst-case targets that should result in maximal unfavorable self-hybridizations of the corresponding gRNAs (Fig. 4). Because the GOLD-tracR enables efficient editing of refractory targets for which the gRNA spacer is perfectly complementary to the 3' end of the tracR and also when the end of the spacer forms a superstable hairpin with itself, we speculate that absent cleavage of any spacer sequence which is due to complementarity to the tracR backbone end or partially with the spacer itself can be solved or ameliorated by the GOLD-tracR. When the spacer is perfectly complementary to the 3' end of the spacer the GOLD-tracR did not increase the moderate genome editing efficiency, which could be due to the inability of the superstable hairpin in the tracR to influence self-folding of the independent crRNA molecule. In such a case a GOLD-sgRNA with a covalent fusion loop between crRNA and GOLD-tracR might help to increase genome editing efficiency. The single case in which no genome editing efficiency could be achieved even when the GOLD-tracR was applied occurred when the spacer sequence is fully self-complementary. Fortunately, this constitutes an extremely rare case underlined by the fact that we had to insert this target in a cell line as it did not exist in the human genome. Nevertheless, such a target might be editable if the spacer is designed to carry deliberate mismatches that prevent self-folding but still allow some cleavage.

While we and others continuously develop tools to increase efficiency of DNA repair pathways choice (Riesenberg et al., 2019; Yeh et al., 2019) few tools are available to increase CRISPR-Cas9 cleavage and therefore general genome editing efficiency. We provide an improved tracR that should increase editing efficiency of most targets and allow cleavage of otherwise non-editable loci. This will reduce the need to pre-screen several gRNAs to find an efficient one for the target of interest and could be especially useful in CRISPR screens of thousands of targets to find genetic perturbations responsible for cellular phenotypes of interest. While it is not possible to incorporate chemical modifications in expressed screen gRNAs, superstable hairpins can be easily added to the CRISPR gRNA library design and thus increase editing efficiency for the whole library. Because misfolding will not only prevent cleavage but also binding of Cas9 to the target, it is probable that the GOLD-tracR scaffold could also be beneficial for other applications in which a Cas9 variant is linked to functional modules like gene activators/repressors or base editors (Adli, 2018). Furthermore, it can be applied to inherently self-complementary prime editing gRNAs or to gRNAs of other CRISPR systems in which a hairpin is amenable to modification.

### References

Adli, M. (2018). The CRISPR tool kit for genome editing and beyond. Nat Commun 9, 1911.
Anderson EM, McClelland S, Maksimova E, Strezoska Ž, Basila M, Briner AE, et al. (2018). Lactobacillus gasseri CRISPR-Cas9 characterization In Vitro reveals a flexible mode of protospacer-adjacent motif recognition. PLoS ONE 13(2): e0192181. https:/Idoi.org/10.1371/journal.pone.0192181.
Babu, K., Amrani, N., Jiang, W., Yogesha, S.D., Nguyen, R., Qin, P.Z., and Rajan, R. (2019). Bridge Helix of Cas9 Modulates Target DNA Cleavage and Mismatch Tolerance. Biochemistry 58, 1905-1917.
Bhagwat, A.M., Graumann, J., Wiegandt, R., Bentsen, M., Welker, J., Kuenne, C., Preussner, J., Braun, T. and Looso, M. (2020). multicrispr: gRNA design for prime editing and parallel targeting of thousands of targets. Life Sci Alliance 9;3 (11):e202000757.
Bellaousov, S., Reuter, J.S., Seetin, M.G., and Mathews, D.H. (2013). RNAstructure: Web servers for RNA secondary structure prediction and analysis. Nucleic Acids Res 41, W471-474.
Briner, A.E., Donohoue, P.D., Gomaa, A.A., Selle, K., Slorach, E.M., Nye, C.H., Haurwitz, R.E., Beisel, C.L., May, A.P., and Barrangou, R. (2014). Guide RNA functional modules direct Cas9 activity and orthogonality. Mol Cell 56, 333-339.
Chen, A.A., and Garcia, A.E. (2013). High-resolution reversible folding of hyperstable RNA tetraloops using molecular dynamics simulations. Proc Natl Acad Sci U S A 110, 16820-16825.
Chen, B., Gilbert, L.A., Cimini, B.A., Schnitzbauer, J., Zhang, W., Li, G.W., Park, J., Blackburn, E.H., Weissman, J.S., Qi, L.S., et al. (2013). Dynamic imaging of genomic loci in living human cells by an optimized CRISPR/Cas system. Cell 155, 1479-1491.
Doench, J.G., Fusi, N., Sullender, M., Hegde, M., Vaimberg, E.W., Donovan, K.F., Smith, I., Tothova, Z., Wilen, C., Orchard, R., et al. (2016). Optimized sgRNA design to maximize activity and minimize off-target effects of CRISPR-Cas9. Nat Biotechnol 34, 184-191.
Graf, R., Li, X., Chu, V.T., and Rajewsky, K. (2019). sgRNA Sequence Motifs Blocking Efficient CRISPR/Cas9-Mediated Gene Editing. Cell Rep 26, 1098-1103 e1093. Hendel, A., Bak, R.O., Clark, J.T., Kennedy, A.B., Ryan, D.E., Roy, S., Steinfeld, I., Lunstad, B.D., Kaiser, R.J., Wilkens, A.B., et al. (2015). Chemically modified guide RNAs enhance CRISPR-Cas genome editing in human primary cells. Nat Biotechnol 33, 985-989.
Jiang, F., Taylor, D.W., Chen, J.S., Kornfeld, J.E., Zhou, K., Thompson, A.J., Nogales, E., and Doudna, J.A. (2016). Structures of a CRISPR-Cas9 R-loop complex primed for DNA cleavage. Science 351, 867-871.
Jinek, M., Chylinski, K., Fonfara, I., Hauer, M., Doudna, J.A., and Charpentier, E. (2012). A programmable dual-RNA-guided DNA endonuclease in adaptive bacterial immunity. Science 337, 816-821.
Kircher, M., Sawyer, S., and Meyer, M. (2012). Double indexing overcomes inaccuracies in multiplex sequencing on the Illumina platform. Nucleic Acids Res 40, e3.
Landrum, M.J., Lee, J.M., Benson, M., Brown, G.R., Chao, C., Chitipiralla, S., Gu, B., Hart, J., Hoffman, D., Jang, W., et al. (2018). ClinVar: improving access to variant interpretations and supporting evidence. Nucleic Acids Res 46, D1062-D1067.
Li, H., Handsaker, B., Wysoker, A., Fennell, T., Ruan, J., Homer, N., Marth, G., Abecasis, G., Durbin, R., and Genome Project Data Processing, S. (2009). The Sequence Alignment/Map format and SAMtools. Bioinformatics 25, 2078-2079.
Makarova, K.S., Wolf, Y.I., Koonin E.V. et al. (2020). Evolutionry classification of CRISPR-Cas systems: a burst of class 2 and derived variants. Nature Reviews Microbiol 18, 67-83.
Meyer, M., and Kircher, M. (2010). Illumina sequencing library preparation for highly multiplexed target capture and sequencing. Cold Spring Harb Protoc 2010, pdb prot5448.
Moreno-Mateos, M.A., Vejnar, C.E., Beaudoin, J.D., Fernandez, J.P., Mis, E.K., Khokha, M.K., and Giraldez, A.J. (2015). CRISPRscan: designing highly efficient sgRNAs for CRISPR-Cas9 targeting in vivo. Nat Methods 12, 982-988.
Mullally, G., van Aelst, K., Naqvi, M.M., Diffin, F.M., Karvelis, T., Gasiunas, G., Siksnys, V., and Szczelkun, M.D. (2020). 5' modifications to CRISPR-Cas9 gRNA can change the dynamics and size of R-loops and inhibit DNA cleavage. Nucleic Acids Res 48, 6811-6823.
Nishimasu, H., Ran, F.A., Hsu, P.D., Konermann, S., Shehata, S.I., Dohmae, N., Ishitani, R., Zhang, F., and Nureki, O. (2014). Crystal structure of Cas9 in complex with guide RNA and target DNA. Cell 156, 935-949.
Pinello, L., Canver, M.C., Hoban, M.D., Orkin, S.H., Kohn, D.B., Bauer, D.E., and Yuan, G.C. (2016). Analyzing CRISPR genome-editing experiments with CRISPResso. Nat Biotechnol 34, 695-697.
Renaud, G., Stenzel, U., and Kelso, J. (2014). leeHom: adaptor trimming and merging for Illumina sequencing reads. Nucleic Acids Res 42, e141.
Richardson, C.D., Ray, G.J., Bray, N.L., and Corn, J.E. (2016). Non-homologous DNA increases gene disruption efficiency by altering DNA repair outcomes. Nat Commun 7, 12463.
Riesenberg, S., Chintalapati, M., Macak, D., Kanis, P., Maricic, T., and Paabo, S. (2019). Simultaneous precise editing of multiple genes in human cells. Nucleic Acids Res 47, e116.
Riesenberg, S., and Maricic, T. (2018). Targeting repair pathways with small molecules increases precise genome editing in pluripotent stem cells. Nat Commun 9, 2164.
Rueda, F.O., Bista, M., Newton, M.D. et al. (2017). Mapping the sugar dependency for rational generation of a DNA-RNA hybrid-guided Cas9 endonuclease. Nat Commun 8, 1610. https://doi.org/10.1038/s41467-017-01732-9.
Scott, T., Urak, R., Soemardy, C. and Morris, K.V. (2019). Improved Cas9 activity by specific modifications of the tracrRNA. Scientific Reports 9, 16104.
Talas, A., Huszar, K., Kulcsar, P.l., Varga, J.K., Varga, E., Toth, E., Welker, Z., Erdos, G., Pach, P.F., Welker, A., et al. (2021). A method for characterizing Cas9 variants via a one-million target sequence library of self-targeting sgRNAs. Nucleic Acids Res. Thyme, S.B., Akhmetova, L., Montague, T.G., Valen, E., and Schier, A.F. (2016). Internal guide RNA interactions interfere with Cas9-mediated cleavage. Nat Commun 7, 11750.
Tuerk, C., Gauss, P., Thermes, C., Groebe, D.R., Gayle, M., Guild, N., Stormo, G., d'Aubenton-Carafa, Y., Uhlenbeck, O.C., Tinoco, I., Jr., et al. (1988). CUUCGG hairpins: extraordinarily stable RNA secondary structures associated with various biochemical processes. Proc Natl Acad Sci U S A 85, 1364-1368.
Varani, G., Cheong, C., and Tinoco, I., Jr. (1991). Structure of an unusually stable RNA hairpin. Biochemistry 30, 3280-3289.
Wang, D., Zhang, C., Wang, B., Li, B., Wang, Q., Liu, D., Wang, H., Zhou, Y., Shi, L., Lan, F., et al. (2019a). Optimized CRISPR guide RNA design for two high-fidelity Cas9 variants by deep learning. Nat Commun 10, 4284.
Wang, J., Williams, B., Chirasani, V.R., Krokhotin, A., Das, R., and Dokholyan, N.V. (2019b). Limits in accuracy and a strategy of RNA structure prediction using experimental information. Nucleic Acids Res 47, 5563-5572.
Wilson, L.O.W., O'Brien, A.R., and Bauer, D.C. (2018). The Current State and Future of CRISPR-Cas9 gRNA Design Tools. Front Pharmacol 9, 749.
Yeh, C.D., Richardson, C.D., and Corn, J.E. (2019). Advances in genome editing through control of DNA repair pathways. Nat Cell Biol 21, 1468-1478.
Yin, H., Song, C.Q., Suresh, S., Wu, Q., Walsh, S., Rhym, L.H., Mintzer, E., Bolukbasi, M.F., Zhu, L.J., Kauffman, K., et al. (2017). Structure-guided chemical modification of guide RNA enables potent non-viral in vivo genome editing. Nat Biotechnol 35, 1179-1187.

## Claims

1. A guide RNA (gRNA) suitable for CRISPR-mediated Oligonucleotide binding and/or editing, comprising:
- a target-specific spacer sequence and
- a constant gRNA sequence comprising at least one locked hairpin secondary structure at a position that does not interfere with Oligonucleotide binding and/or editing.

2. The gRNA of claim 1 comprising:
- a target-specific spacer sequence and
- a constant gRNA sequence comprising a CRISPR repeat/anti-repeat-structure or a portion thereof and at least one locked hairpin secondary structure at a position that does not interfere with Oligonucleotide binding and/or editing.

3. The gRNA of claim 1 or 2 comprising in 5' to 3' direction:
- a target-specific spacer sequence and
- a constant gRNA sequence comprising in 5' to 3' direction a (i) repeat/anti-repeat structure, (ii) optionally a nexus sequence and (iii) at least one locked hairpin secondary structure.

4. The gRNA of any one of claims 1-3 comprising in 5' to 3' direction a target-specific spacer sequence, a repeat sequence, an anti-repeat sequence, optionally a nexus, and a hairpin sequence comprising a first hairpin and optionally a second hairpin and wherein at least the first hairpin has a locked secondary structure.

5. The gRNA of any one of claims 1-4, which (i) consists of a single RNA molecule, particularly a single guide RNA (sgRNA) molecule, or which (ii) comprises at least two RNA molecules, particularly a spacer-containing CRISPR RNA (crRNA) molecule and a trans-activating CRISPR RNA (tracrRNA) molecule.

6. The gRNA of any one of claims 1-5, wherein
(i) the locked hairpin forms a secondary structure comprising a contiguous stem having a length of at least 6 nt and particularly forms a secondary structure comprising a contiguous stem having a length of 6, 7, 8, 9 or 10 nt, more particularly of 8 nt, and/or
(ii) the locked hairpin forms a secondary structure comprising a contiguous stem having at least 2 C-G base pairs and particularly forms a secondary structure comprising a contiguous stem having 2, 3, 4 or 5 C-G base pairs, particularly 4 C-G base pairs.

7. The gRNA of any one of claims 1-6, which comprises at least one modified nucleotide building block, particularly at least one modified nucleotide building block selected from:
(a) a nucleobase-modified building block,
(b) a sugar-modified building block,
(c) a backbone-modified building block,
(d) a modified nucleotide building block having attached thereto a heterologous moiety, and
(e) any combination thereof.

8. The gRNA of any one of claims 1-7, wherein the locked hairpin comprises
(i) the nucleotide sequence SEQ ID NO.1:
5'-X₁-XₙGGAC(N)ᵣGUCCXₙ₊₁-X₂ₙ-3'
wherein X is any nucleotide e.g. selected from A, C, G, or U, with the proviso that X₁-Xₙ form a contiguous hairpin stem with Xₙ₊₁-X₂ₙ and n is 2, 3, 4, 5, or 6, particularly 3, 4 or 5, and more particularly 4; and
Nᵣ is the loop of the first hairpin; and N is any nucleotide e.g. selected from A, C, G, or U, and Nᵣ is particularly selected from 5'-GNRA-3', 5'-UNCG-3' and 5'- CUUG-3',wherein R is a nucleotide selected from A or G, and N is any nucleotide, e.g. selected from A, C, G, or U, and r is 3, 4, 5 or 6, particularly 4 or 5, and more particularly 4;
(ii) the nucleotide sequence SEQ ID NO. 2:
5' -ACUUGGAC(N)rGUCCAAGU-3'
wherein Nᵣ is the loop of the locked hairpin, N is any nucleotide particularly selected from A, C, G, or U, and Nᵣ is particularly selected from 5'-GNRA-3', 5'-UNCG-3' and 5'- CUUG-3',wherein R is a nucleotide selected from A or G, and N is any nucleotide, e.g. selected from A, C, G, or U, and r is 3, 4, 5 or 6, particularly 4 or 5, and more particularly 4; or
(iii) the nucleotide sequence SEQ ID NO. 3:
5'-ACUUGGACUUCGGUCCAAGU-3'.

9. The gRNA according to any one of claims 1-8, comprising
(i) the nucleotide sequence SEQ ID NO. 4: 
5’-
AGCAUAGCAAGUUAAAAUAAGGCUAGUCCGUUAUCAACUUGGACUUCGGUCC
AAGUGGCACCGAGUCGGUGCUUU-3’
 wherein A, C, G, and U are nucleotide building blocks including modified nucleotide building blocks, or
(ii) the nucleotide sequence SEQ ID NO. 5: 
5’-
mA*mG*mCmAmUmAmGmCmAmAGUUmAAmAAUAmAmGGCUAGUCmCGUUAm
UmCAAmCmUmUGGACUUCGGUCCmAmAmGmUmGGmCmAmCmCmGmAmGm
UmCmGmGmUmGmCmU*mU*mU-3’
 wherein A, C, G, and U are non-modified nucleotide building blocks, mA, mC, mG, and mU are sugar-modified nucleotide building blocks, particularly 2'-O-Methyl-modified building blocks and * denotes a modified internucleosidic linkage, particularly a phosphorothioate linkage.

10. A trans-activating CRISPR RNA (tracrRNA) suitable for CRISPR-mediated Oligonucleotide binding and/or editing comprising a constant gRNA sequence comprising at least one locked hairpin secondary structure at a position that does not interfere with Oligonucleotide binding and/or editing, which particularly comprises at least one of the features as defined in any one of claims 2-9.

11. A nucleic acid molecule encoding a gRNA of any one of claims 1-9 or a tracrRNA of claim 10 optionally in operative linkage with an expression control sequence.

12. A library comprising a plurality of gRNAs of any one of claims 1-9 comprising different target-specific spacer sequences and a constant gRNA backbone.

13. A complex comprising a Cas enzyme and a gRNA of any one of claims 1-9 or a Cas enzyme and a tracrRNA of claim 10.

14. *In vitro* or *ex vivo* use of a gRNA of any one of claims 1-9, a tracrRNA of claim 10, a gRNA- or tracrRNA-encoding nucleic acid molecule of claim 11, a library of claim 12 or a complex of claim 13 for CRISPR-mediated Oligonucleotide binding and/or editing, particularly for genome editing.

15. A preparation comprising gRNA of any one of claims 1-9, a tracrRNA of claim 10, a gRNA- or tracrRNA-encoding nucleic acid molecule of claim 11, a library of claim 12 or a complex of claim 13 for use in therapy including human medicine and veterinary medicine.
